(19) Europäisches Patentamt
European Patent Office
Office européen des brevets

(11) **EP 2 510 956 B1**

(12) **EUROPEAN PATENT SPECIFICATION**

(45) Date of publication and mention of the grant of the patent:
**30.08.2017 Bulletin 2017/35**

(51) Int Cl.:
*A61L 27/00* (2006.01)  *A61K 35/12* (2015.01)
*A61K 35/28* (2015.01)  *A61K 35/34* (2015.01)
*A61K 38/00* (2006.01)  *A61P 9/04* (2006.01)
*A61P 9/10* (2006.01)  *C12N 5/077* (2010.01)
*C12N 5/071* (2010.01)  *A61L 27/38* (2006.01)
*A61K 35/35* (2015.01)  *A61L 27/36* (2006.01)

(21) Application number: **10834436.7**

(22) Date of filing: **08.10.2010**

(86) International application number:
**PCT/JP2010/067785**

(87) International publication number:
**WO 2011/067983 (09.06.2011 Gazette 2011/23)**

(54) **ADIPOCYTE SHEET, THREE-DIMENSIONAL STRUCTURE THEREOF, AND METHOD FOR PRODUCING THE SAME**

FETTZELLENSCHICHT, DREIDIMENSIONALE STRUKTUR DARAUS UND HERSTELLUNGSVERFAHREN DAFÜR

FEUILLE DE CELLULES ADIPEUSES, STRUCTURE TRIDIMENSIONNELLE DE CELLE-CI, ET PROCÉDÉS DE FABRICATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2009 JP 2009275905**

(43) Date of publication of application:
**17.10.2012 Bulletin 2012/42**

(73) Proprietors:
• **CellSeed Inc.**
  **Tokyo 162-0056 (JP)**
• **Osaka University**
  **Suita-shi**
  **Osaka 565-0871 (JP)**

(72) Inventors:
• **IMANISHI, Yukiko**
  **Suita-shi**
  **Osaka 565-0871 (JP)**
• **SAWA, Yoshiki**
  **Suita-shi**
  **Osaka 565-0871 (JP)**
• **SHIMOMURA, Iichiro**
  **Suita-shi**
  **Osaka 565-0871 (JP)**
• **MAEDA, Norikazu**
  **Suita-shi**
  **Osaka 565-0871 (JP)**
• **MIYAGAWA, Shigeru**
  **Suita-shi**
  **Osaka 565-0871 (JP)**
• **SAKAI, Hideaki**
  **Tokyo 162-0056 (JP)**

(74) Representative: **Vossius & Partner**
**Patentanwälte Rechtsanwälte mbB**
**Siebertstrasse 3**
**81675 München (DE)**

(56) References cited:
**WO-A1-2006/080434    JP-A- 2007 528 755**
**US-A1- 2005 261 362**

EP 2 510 956 B1

**(Cont. next page)**

- Yasuhiro Shudo ET AL: "Heart Failure: Cell and Gene Based Therapies I Abstract 3258: A Combined Cell Sheets With Adipose Tissue Derived Mesenchymal Stem Cells and Skeletal Myoblasts Provide Complete Myocardial Regeneration in Rat Myocardial Infarction Model -- Shudo et al. 120 (10018): S773 -- Circulation", Circulation, 3 November 2009 (2009-11-03), XP055115223, Retrieved from the Internet: URL:http://circ.ahajournals.org/cgi/conten t/meeting_abstract/120/18_MeetingAbstracts /S773-b [retrieved on 2014-04-24]
- SHIBATA R ET AL: "Usefulness of Adiponectin to Predict Myocardial Salvage Following Successful Reperfusion in Patients With Acute Myocardial Infarction", AMERICAN JOURNAL OF CARDIOLOGY, CAHNERS PUBLISHING CO., NEWTON, MA, US, vol. 101, no. 12, 15 June 2008 (2008-06-15), pages 1712-1715, XP022710417, ISSN: 0002-9149, DOI: 10.1016/J.AMJCARD.2008.02.057 [retrieved on 2008-04-09]
- EREN, P. ET AL.: 'Adiponectinemia controls pro-angiogenic cell therapy' STEM CELLS vol. 27, no. 11, 25 September 2009, pages 2712 - 2721, XP008153356
- SHIBATA, R. ET AL.: 'Adiponectin and cardiovascular disease' CIRC J vol. 73, no. 4, 03 March 2009, pages 608 - 614, XP008153396
- SUMI, M. ET AL.: 'Transplantation of adipose stromal cells, but not mature adipocytes, augments ischemia-induced angiogenesis' LIFE SCI vol. 80, no. 6, 2007, pages 559 - 665, XP008153354
- YASUHIRO SHUDO ET AL.: 'Kingasaibo to Shibo Yurai Kan'yokei Kansaibo o Heiyo shita Saibo Sheet ni yoru Rat Manseiki Shinkin Kosoku Model ni Taisuru Shinkin Saisei Koka no Kento' GEN THORAC CARDIOVASC SURG vol. 57, 10 September 2009, page 488, XP008153442

**Description**

Technical Field

The present invention relates to an adipocyte sheet and a three-dimensional structure thereof, which are applicable to the heart. More specifically, the present invention relates to a cell sheet containing adult adipocytes, applicable to the heart, and useful in the fields of medicine, biology, drug discovery, pharmaceuticals, and the like; a method for producing the same; and a method for using the same.

Background Art

Myocardial infarction is an irreversible damage (Non-Patent Literature 1). Ischemic heart diseases are the cause of 50% of all cardiovascular system-related deaths, and are the main cause of congestive cardiac failure. Among patients diagnosed with congestive cardiac failure, the one-year mortality rate as a result of chronic cardiac disease is 20% (Non-Patent Literature 2). Most of the treatments currently available to the clinician can significantly improve the prognosis of patients with acute myocardial infarction. Although angioplasty and thrombolytic agents can remove the cause of acute myocardial infarction, the period from the onset of occlusion to reperfusion determines the degree of irreversible myocardial damage (Non-Patent Literature 3). No clinically used drugs or treatment is effective in replacing myocardial scars with functional contractile tissue. There is a demand for a novel treatment for regenerating normal cardiomyocytes.

Although cardiomyoplasty has been proposed as a surgical method to improve left ventricular (LV) function in patients with congestive cardiac failure, the effect thereof on cardiac function is still unclear (Non-Patent Literature 4 and 5). In recent years, implantation of a biologically engineered cardiac graft in which a biodegradable scaffold is used has been proposed as another novel strategy. However, this strategy shows only minimal benefit in improving cardiac function because such a graft achieves almost no attachment to the myocardium (Non-Patent Literature 6 and 7). The key to the regeneration of damaged myocardium may be the ability of biologically engineered cardiac tissue to histologically and electrically integrate with the recipient heart.

It has long been reported that immunological rejection occurs when exogenous tissue, such as an allogeneic graft (or allograft) or a xenograft, is used in the transplantation of organs (e.g., heart, blood vessel, etc.) (Non-Patent Literature 8 and 9). Rejection of arterial grafts pathologically causes graft dilation (which leads to rupture) or occlusion. The former is caused by the degradation of extracellular matrices, while the latter is caused by cell growth in a blood vessel (Non-Patent Literature 10).

In recent years, cell transplantation has been drawing attention as a treatment method that uses a biological material. However, transplantation of human myoblasts into an infarcted heart has the following drawbacks: 1. damage and loss of transplanted cells; 2. tissue damage in the recipient heart during injection; 3. tissue supply efficiency to the recipient heart; 4. the occurrence of arrhythmia; and 5. difficulty in treating the entire infarction site. Therefore, cell transplantation methods must be further improved.

Adiponectin has recently been the subject of attention because of the possibility of protecting the myocardium after injury and preventing exacerbation of cardiac function by actions against apoptosis, inflammation, fibrosis, and myocardial hypertrophy, and action for angiogenesis (Non-Patent Literature 11 and 12). However, adiponectin administration must continue for a long period of time in order to confirm the effect. Repeated injections to myocardial tissue are highly invasive and difficult at the practical level. It is also not practical because an immense amount of protein is necessary in the case of systemic administration, and only a slight amount of protein reaches the desired tissue when the protein is injected into the blood because the protein is susceptible to degradation (Non-Patent Literature 13). Further, methods of increasing the serum adiponectin level by drugs such as thiazolidine and calorie restriction are not always easy to perform on cardiac failure patients. Countermeasures in such a case include a drug delivery system (DDS) as a conventional technique. However, with existing DDS, it is difficult to achieve sustained release of protein retaining its activity, at an appropriate concentration for a long period of time. Additionally, there is the possibility that inflammation would be caused depending on the drug carrier.

Against such a background, Patent Literature 1 discloses a novel method for culturing cells in which the cells are cultured on a cell culture support having a substrate surface coated with a polymer whose upper or lower critical solution temperature in water is 0 to 80°C, at an upper critical solution temperature or below or at a lower critical solution temperature or above, and subsequently, the temperature of the cell culture medium is brought to the upper critical solution temperature or above or to the lower critical solution temperature or below so as to peel off the cultured cells without enzyme treatment. Further, according to the disclosure of Patent Literature 2, the above-described temperature-responsive cell culture substrate is used to culture cardiomyocytes at the upper critical solution temperature or above or at the lower critical solution temperature or below, and subsequently, the temperature of the mediumis brought to the upper critical solution temperature or above or to the lower critical solution temperature or below, thereby peeling off the cultured cardiomyocytes with minimum damage. Various new developments have been introduced into conventional culture techniques by the use of temperature-responsive cell culture substrates. In Patent Literature 3, such a technique was further developed, and it was found that although it had been difficult to improve cardiac function with conventional techniques, it is possible to improve cardiac function over an extended period of time by using myoblasts other than cardiac tissue as a cell sheet. Further, Patent Literature 4 found that the cardiac function can be improved by using mesenchymal stem cells as a cell sheet. Further treatment effect can be expected with further improvement in the function of these cell sheets, and there are expectations for further development.

Citation List

Patent Literature

PLT 1: Japanese Unexamined Patent Publication No. H02-211865
PLT 2: Japanese Domestic Re-publication of PCT International Publication No. 2002-08387
PLT 3: Japanese Domestic Re-publication of PCT International Publication No. 2005-011524
PLT 4: Japanese Domestic Re-publication of PCT International Publication No. 2006-080434

Non-Patent Literature

NPL 1: Ho KK, Anderson KM, Kannel WB, Grossman W, Levy D., Circulation. 1993; 88: 107-115
NPL 2: American Heart Association., Dallas, Tex: American Heart Association; 2001
NPL 3: Ryan TJ, Antman EM, Brooks NH, Califf RM, Hillis LD, Hiratzka LF, Rapaport E, Riegel B, Russell RO, Smith EE III, Weaver WD, Gibbons RJ, Alpert JS, Eagle KA, Gardner TJ, Garson A Jr., Gregoratos G, Ryan TJ, Smith SC Jr., J Am Coll Cardiol. 1999; 34: 890-911
NPL 4: Corin WJ, George DT, Sink JD et al., J Thorac Cardiovasc Surg 1992; 104: 1662-71; Kratz JM, Johnson WS, Mukherjee R et al., J Thorac Cardiovasc Surg 1994; 107: 868-78; Carpentier A, Chachques JC., Lancet. 1985; 8840: 1267
NPL 5: Hagege AA, Desnos M, Chachques JC et al., Preliminary report: follow-up after dynamic cardiomyoplasty. Lancet. 1990; 335: 1122-4
NPL 6: Leor J, Etzion SA, Dar A et al., Circulation 2000; 102 [suppl III] III-56-III-61
NPL 7: Li RK, Jia ZQ, Weisel RD et al., Circulation 1999; 100 [suppl II]: II-63-II-69
NPL 8: Carrel A., 1907, J Exp Med 9: 226-8; Carrel A., 1912., J Exp Med 9:389-92; Calne RY., 1970, Transplant Proc 2: 550
NPL 9: Auchincloss 1988, Transplantation 46: 1
NPL 10: Uretsky BF, Mulari S, Reddy S, et al., 1987, Circulation 76: 827-34
NPL 11: Shibata, R., N. Ouchi, and T. Murohara, Adiponectin and cardiovascular disease. Circ J, 2009. 73(4): pp. 608-14
NPL 12: Eren P., Camus S., Matrone G., et al., Adiponectinemia controls pro-angiogenic cell therapy. 2009, Stem Cells 27 (11): pp. 2712-21
NPL 13: Hwang H., Kloner RA. Improving regenerating potential of the heart after myocardial infarction: Factor-based approach. 2010, Life Sciences 86: pp. 461-472

Summary of Invention

Technical Problem

An object of the present invention is to provide an artificial tissue or sheet that tolerates implantation and that can be used in an actual operation and produced by culturing. Another object of the present invention is to provide a novel treatment method as an alternative to cell therapies. In particular, the present invention aims to create an artificial tissue capable of tolerating implantation, using adipocytes as materials. Heretofore, there has been a problem with the use of cultured cells, such as adipocytes, that underwent terminal differentiation, because it has been difficult to maintain the cell survival rate by commonly used enzyme treatment methods when collecting such cultured cells. In the present invention, obtained adipocytes are seeded onto temperature-responsive culture substrate and collected in a sheet form without enzyme treatment, thereby allowing the sheet to be implanted into an affected site in a minimally invasive and highly efficient manner, while maintaining the survival rate of the adipocytes. This allows the grafting of adipocytes at the affected site, and a local and sustainable supply of adiponectin for a long period of time.

Solution to Problem

The present inventors conducted investigations from various angles for research and development in order to solve the above-described problems. As a result, the present inventors found that the use of a cell sheet containing adipocytes and a three-dimensional structure thereof unexpectedly facilitates the development of tissue at the graft site. They also found an artificial tissue that can be easily peeled off from a culture dish. The present invention is completed based on such findings.

Specifically, the present invention provides a cell sheet at least containing adipocytes and a three-dimensional structure thereof, which are applicable to heart disease. The present invention also provides a method for preparing the cell sheet and the three-dimensional structure thereof on a substrate surface coated with a temperature-responsive polymer. The present invention is considered to be an extremely important invention that has been achieved for the first time with the use of a cell structure, based on an absolutely unique and novel idea, i.e., a cell sheet at least containing adipocytes for application to heart disease.

Specifically, the present invention encompasses the following:

Item 1. A graft material for treating heart disease, comprising a cell sheet containing adipocytes.

Item 2. The graft material for treating heart disease according to Item 1, wherein the cell sheet has a thickness of 50 μm or more.

Item 3. The graft material for treating heart disease according to Item 1 or 2, wherein the adipocytes sheet has an adiponectin secretory capacity of $3 \times 10^{-14}$ g/cell or more per day.

Item 4. The graft material for treating heart disease according to any one of Items 1 to 3, wherein the cell sheet has at least one function selected from the group consisting of fibroblast-inhibiting function, angiogenic function, apoptosis-inhibiting function, and anti-inflammatory function.

Item 5. The graft material for treating heart disease according to any one of Items 1 to 4, wherein the adipocytes are obtained by inducing differentiation of adipose derived fibroblasts.

Item 6. The graft material for treating heart disease according to any one of Items 1 to 5, comprising the cell sheet in the form of a three-dimensional structure.

Item 7. The graft material for treating heart disease according to any one of Items 1 to 6, comprising multiple cell sheets in a laminated state.

Item 8. The graft material for treating heart disease according to any one of Items 1 to 7, wherein at least one of the cell sheets contains 50% or more adipocytes.

Item 9. The graft material for treating heart disease according to any one of Items 1 to 7, wherein at least one of the cell sheets contains 60% or more adipocytes.

Item 10. The graft material for treating heart disease according to any one of Items 1 to 7, wherein at least one of the cell sheets contains 70% or more adipocytes.

Item 11. The graft material for treating heart disease according to any one of Items 1 to 7, wherein at least one of the cell sheets contains 80% or more adipocytes.

Item 12. The graft material for treating heart disease according to any one of Items 1 to 7, wherein at least one of the cell sheets contains 90% or more adipocytes.

Item 13. The graft material for treating heart disease according to any one of Items 1 to 7, wherein at least one of the cell sheets contains 95% or more adipocytes.

Item 14. The graft material for treating heart disease according to any one of Items 1 to 13, wherein at least one of the cell sheets further comprises at least one selected from the group consisting of myoblasts and mesenchymal stem cells.

Item 15. The graft material for treating heart disease according to Item 14, wherein the myoblast is a skeletal myoblast.

Item 16. The graft material for treating heart disease according to Item 14 or 15, wherein the mesenchymal stem cell is derived from adipose tissue.

Item 17. The graft material for treating heart disease according to any one of Items 1 to 16, comprising no scaffold.

Item 18. The graft material for treating heart disease according to any one of Items 1 to 17, wherein the graft material is applied to an individual from which the adipocytes are derived.

Item 19. The graft material for treating heart disease according to any one of Items 1 to 18, further comprising a cytokine or growth factor.

Item 20. The graft material for treating heart disease according to any one of Items 1 to 19, wherein the heart disease is at least one disease or disorder selected from the group consisting of cardiac failure, ischemic heart disease, myocardial infarction, cardiomyopathy, myocarditis, hypertrophic cardiomyopathy, dilated phase of hypertrophic cardiomyopathy, and dilated cardiomyopathy.

Item 21. The graft material for treating heart disease according to any one of Items 1 to 20, wherein the cell sheet is isolated from a cell culture support.

Item 22. A method for producing a graft material for treating heart disease comprising a cell sheet containing adipocytes, without using a scaffold, the method comprising the steps of:

a) culturing a cell group containing adipocytes on a cell culture support coated with a temperature-responsive polymer whose upper critical solution temperature or lower critical solution temperature in water is 0 to 80°C, in a culture solution;

b) bringing the temperature of the culture solution to the upper critical solution temperature or above or the lower critical solution temperature or below; and

c) peeling off the cell group as a cell sheet from the cell culture support.

Item 23. The method according to Item 22, further comprising step d) of adding an ascorbic acid or a derivative thereof to the culture solution before step c).

Item 24. The method according to Item 22 or 23, comprising no step of protease treatment.

Item 25. The method according to any one of Items 22 to 24, wherein the temperature-responsive polymer is poly(N-isopropylacrylamide).

Advantageous Effects of Invention

The use of a cell sheet containing adipocytes and a three-dimensional structure thereof, which are provided by the present invention, makes it possible to significantly improve cardiac function compared to when a conventional technique, i.e., a cell sheet consisting of myoblasts or mesenchymal stem cells, is used. The present invention also increases the strength of the cell sheet itself and provides improved grafting procedures.

Brief Description of Drawings

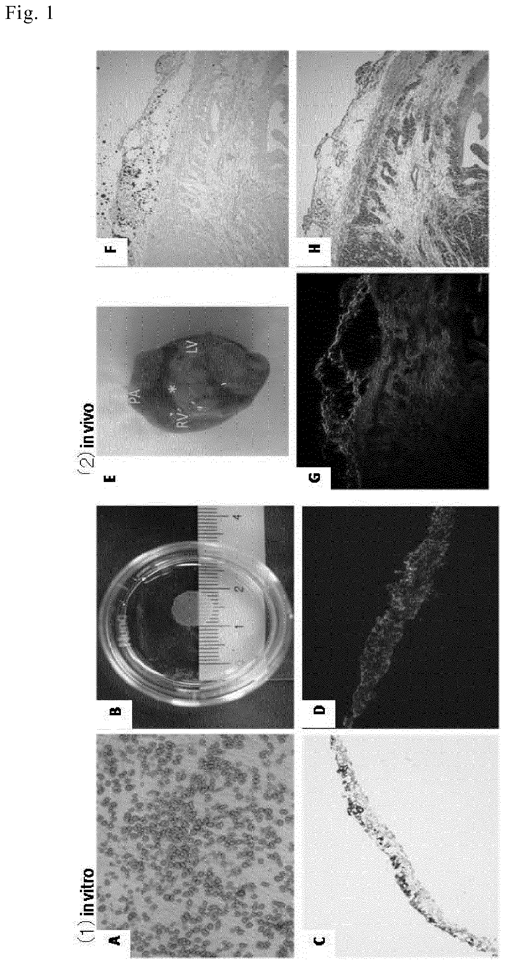

Fig. 1 shows the results of histological examination of adipocyte sheets obtained in Example 1.

(1) *In vitro*

1A. Adipocytes after cultivation and differentiation induction. Differentiation into adipocytes was confirmed by oil red O staining. In the figure, those in the spherical shape are lipid droplets.

1B. Adipocytes were collected in a sheet form using a temperature-responsive culture dish.

1C. Staining with AS oil red O. A number of positive cells were confirmed. In the figure, those in the spherical shape are lipid droplets.

1D. Immunostaining with AS adiponectin. The expression of adiponectin was observed. The light portion in the figure indicates adiponectin.

(2) *In vivo*

1E. Extracted cardiac tissue 28 days after AS implantation in a rat model during acute myocardial infarction. Adhesion of adipocyte sheet-like tissue was observed.

1F. Oil red O staining 28 days after AS implantation in a rat model during acute myocardial infarction. Oil red O positive cell layer engraftment was observed in the epicardial side. The light portion in the figure indicates lipid droplets.

1G. An adiponectin immunostaining image of the same section as F. The expression of adiponectin was observed in the epicardial side and myocardial infarction site. The light portion in the figure is adiponectin.

1H. A hematoxylin-eosin staining image of the same section as F.

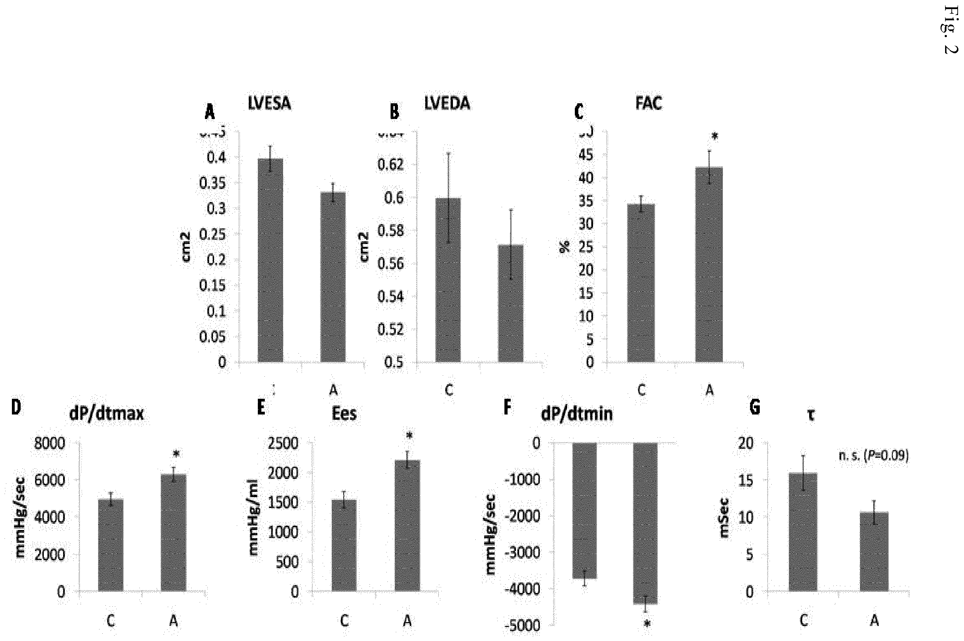

Fig. 2 shows the results of the analysis regarding an effect on cardiac function four weeks after adipocyte sheet implantation during acute myocardial infarction in Example 1.

2A. Left ventricular end-systolic area (LVESA). A tendency to suppress enlargement of the left ventricle by AS implantation was observed.

2B. Left ventricular end-diastolic area (LVEDA). A tendency to suppress enlargement of the left ventricle by AS implantation was observed.

2C. Fractional area change (FAC) in the left ventricle. Left ventricular contractility deterioration was significantly suppressed by AS implantation.

2D. Left ventricular maximum positive dP/dt ($dP/dt_{max}$). Contractile force deterioration was significantly suppressed by AS implantation.

2E. Left ventricular end-systolic elastance (Ees). Contractile force deterioration was significantly suppressed by AS implantation.

2F. Left ventricular maximum negative dP/dt ($dP/dt_{min}$). Expansive force deterioration was significantly suppressed by AS implantation.

2G. Left ventricular isovolumetric relaxation time ($\tau$). A tendency to suppress the deterioration of expansive force by AS implantation was observed.

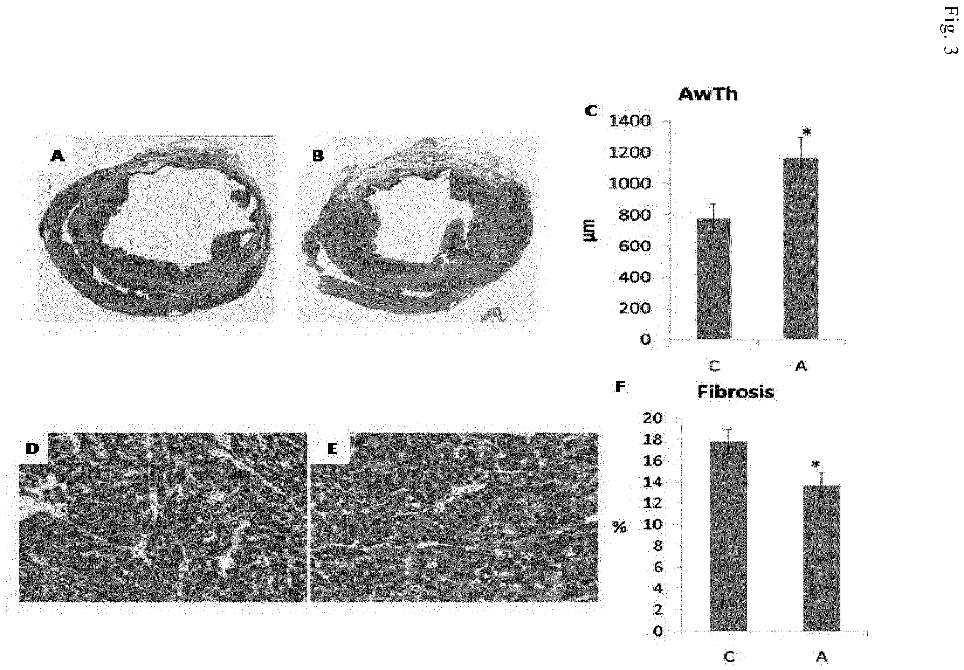

Fig. 3 shows the results of the analysis regarding an effect on myocardial tissue four weeks after adipocyte sheet implantation during acute myocardial infarction in Example 1.

3A. A macro image in Group C.

3B. A macro image in Group A. Compared to Group C, suppression of the thinning of the left ventricular anterior wall was observed.

3C. The thickness of the left ventricular anterior wall in groups C and A was calculated. The thinning of the left ventricular anterior wall was significantly suppressed by AS implantation. 3D. An enhanced enlarged image at the boundary between the infarction site and the normal site in Group C.

3E. An enhanced enlarged image at the boundary between the infarction site and the normal site in Group A. Compared to Group C, fibrosis was alleviated.

3F. The fibrotic ratio at the boundary between the infarction site and the normal site in Groups C and A was measured. Fibrosis was significantly suppressed by AS implantation.

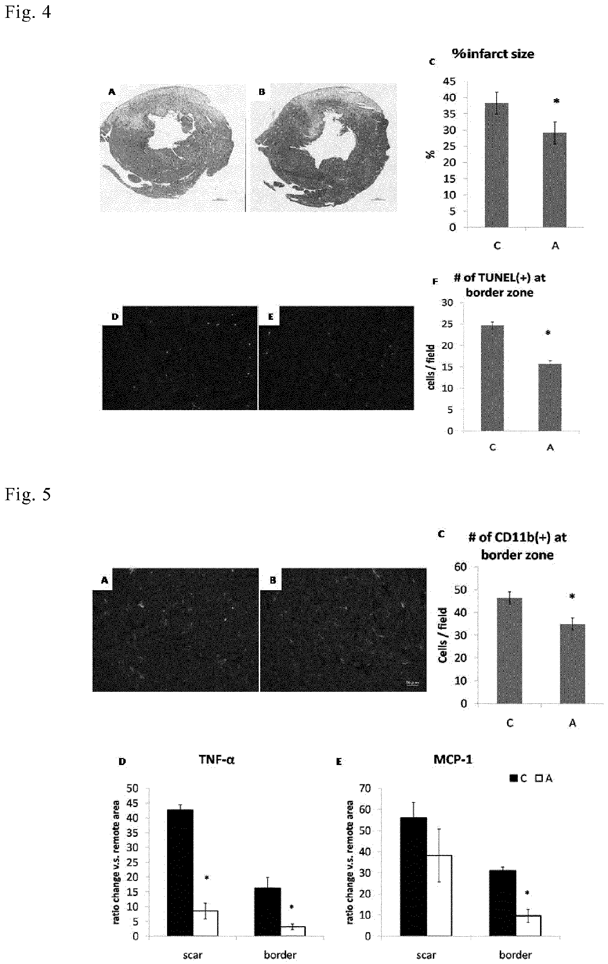

Fig. 4 shows the results of the analysis regarding a myocardial protection effect two days after adipocyte sheet implantation in Example 1.

4A. A macro image of hematoxylin-eosin staining in Group C.

4B. A macro image of hematoxylin-eosin staining in Group A. Compared to Group C, the infarction range was reduced.

4C. The infarction rate in Groups C and A (the proportion of the distance of the infarction site to the entire left ventricle circumference). The infarction range was significantly reduced by AS implantation.

4D. A TUNEL chromatic image at the boundary between the infarction site and the normal site in Group C.

4E. A TUNEL chromatic image at the boundary between the infarction site and the normal site in Group A. Compared to Group C, the number of TUNEL positive cells was reduced. The light portion in the figure indicates TUNEL positive cells.

4F. The number of TUNEL positive cells in Groups C and A was quantified. The number of apoptosis cells was significantly reduced by adipocyte sheet implantation.

Fig. 5 shows the results of the analysis regarding an anti-inflammation effect by adipocyte sheet implantation in Example 1.

5A. A CD11b immunostaining image at the boundary between the infarction site and the normal site in Group C.

5B. A CD11b immunostaining image at the boundary between the infarction site and the normal site in Group A. Compared to Group C, the number of CD11b positive cells was reduced. The light portion in the figure indicates CD11b positive cells.

5C. The number of CD11b positive cells in Groups C and A was quantified. The number of infiltration macrophage was significantly reduced by AS implantation.

5D. The transcript amounts of TNF-$\alpha$ at the infarction site and the boundary between the infarction site and the normal site were quantified. Tumor necrosis factor alpha (TNF-$\alpha$) transcript was suppressed by AS implantation.

5E. The transcript amounts of Monocyte Chemotactic Protein-1 (MCP-1) at the infarction site and the boundary between the infarction site and the normal site were quantified. MCP-1 transcript was suppressed by AS implantation.

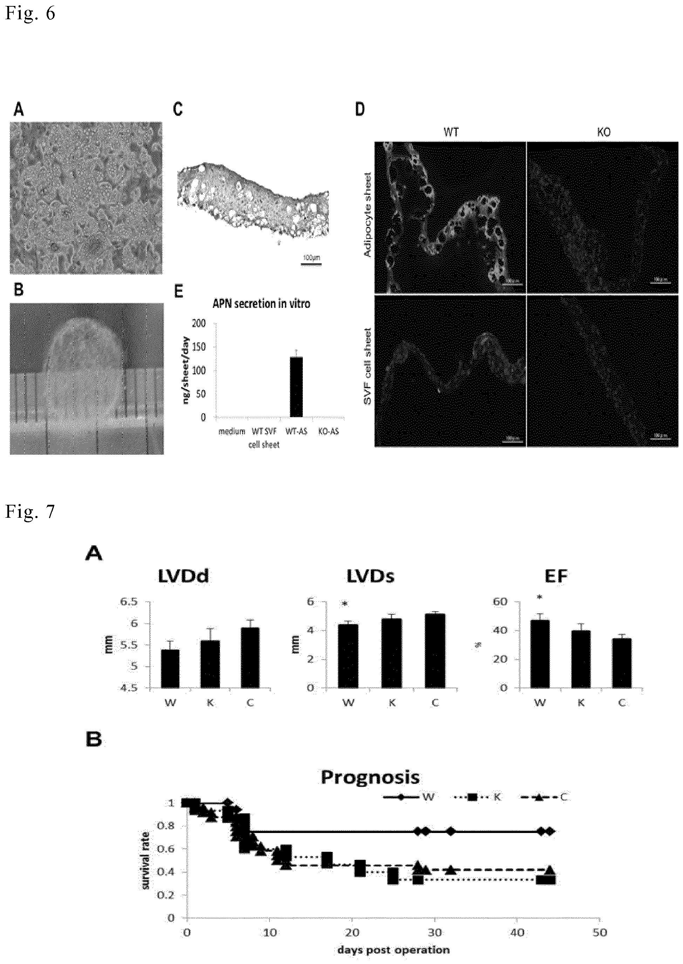

Fig. 6 shows the result of the histological examination of adipocyte sheets in Example 2.

6A. Adipocytes after cultivation and differentiation induction. Differentiation into adipocytes was confirmed.

6B. Adipocytes were collected in a sheet form using a temperature-responsive culture dish. The diameter of the adipocyte sheet was about 7 mm.

6C. A hematoxylin-eosin staining image of the adipocyte cell sheet. The thickness was about 100 $\mu$m.

6D. Adiponectin immunostaining of adipocyte sheet. Adiponectin was expressed in the wild-type mouse-derived (WT) adipocyte sheet. (The light portion in the figure indicates adiponectin.) Adiponectin was not expressed in the wild-type (WT) preadipocyte sheet (SVF cell sheet), adiponectin knockout mouse (KO)-derived adipocyte sheet, and preadipocyte sheet.

6E. The amount of adiponectin in a cell sheet culture supernatant. Adiponectin was detected in the culture supernatant of a wild-type mouse-derived adipocyte sheet (WT-AS). Adiponectin was not detected in the culture medium, and the culture supernatants of the wild-type mouse-derived preadipocyte sheet (WT-SVF cell sheet) and adiponectin knockout mouse-derived adipocyte sheet (KO-AS).

Fig. 7 shows the results of the analysis regarding an effect on cardiac function four weeks after adipocyte sheet implantation during acute myocardial infarction in Example 1.

7A. Left ventricle end-diastolic dimension (LVDd). In the wild-type mouse-derived adipocyte sheet implantation group (W), a tendency to suppress the enlargement of the left ventricle was observed compared to the adiponectin knockout mouse-derived adipocyte sheet implantation group (K) and the untreated group (C). Left end-systolic dimension (LVDs). In Group W, enlargement of the left ventricle was significantly suppressed compared to Group C. A tendency to suppress enlargement of the left ventricle was observed, although it was not so significant compared to Group K. Left ventricle ejection rate (EF). In Group W, the left ventricle ejection rate was significantly increased compared to Group C. A tendency to increase the left ventricle ejection rate was observed, although it was not so significant compared to Group K.

7B. Life prognosis. In Group W, life expectancy was significantly extended compared to Group K and Group C.

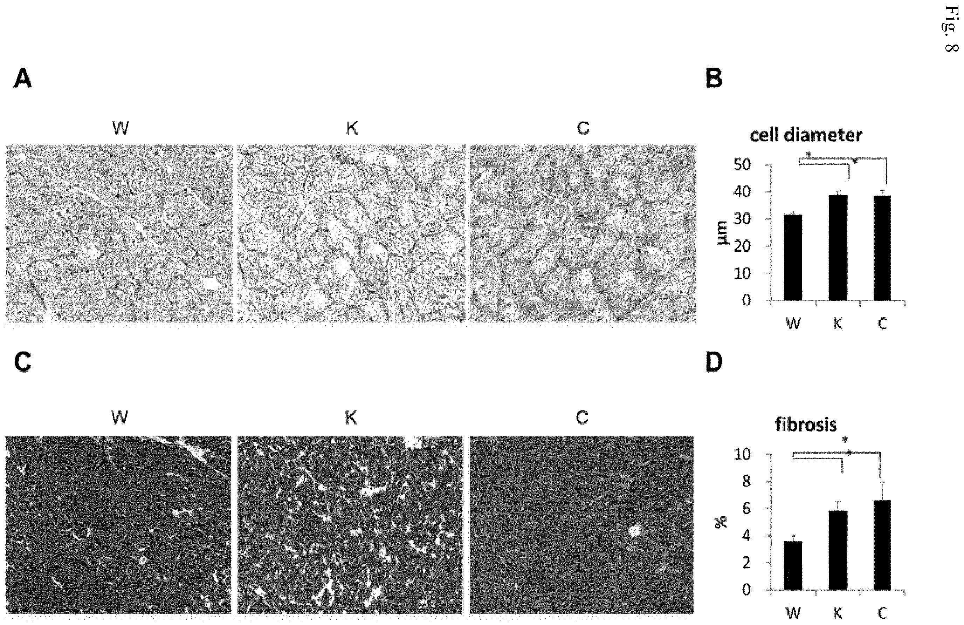

Fig. 8 shows the results of the analysis regarding an effect on myocardial tissue four weeks after adipocyte sheet implantation during acute myocardial infarction in Example 1.

8A. A typical tissue image of periodic acid-Schiff (PAS) staining. When a myocardial infarction distant site was examined using a microscope, cardiomyocyte hypertrophy was observed in Group C. In Group K, left ventricular hypertrophy similar to that in Group C was observed. In Group W, cardiomyocyte hypertrophy was suppressed compared to the other two groups. The dark portion in the figure indicates cardiac sarcolemma.

8B. The cardiomyocyte diameter in the myocardial infarction distant site in each experimental group was measured. In Group W, cardiomyocyte hypertrophy was significantly suppressed compared to the other two groups.

8C. A typical tissue image of Masson trichrome staining. When the boundary between the myocardial infarction site and the normal site was observed using a microscope, Group C showed appropriate fibrosis. In Group K, fibrosis similar to that in Group C was observed. In Group W, fibrosis was reduced compared to the other two groups.

8D. The fibrosis level at the boundary between the myocardial infarction site and the normal site was calculated. In Group W, fibrosis was significantly suppressed compared to the other two groups.

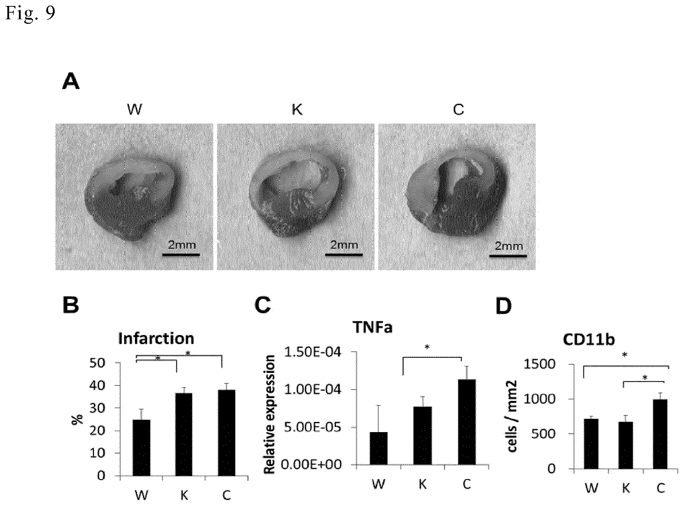

Fig. 9 shows the results of the analysis regarding a myocardial protection effect two days after the adipocyte sheet implantation treatment in Example 2.

9A. A typical tissue image of the left ventricular short-axis section by 2,3,5-triphenyltetrazolium chloride (TTC) staining. In Group W, the infarction area was smaller than the other two groups.

9B. The proportion of the infarction area to the entire area of the left ventricle in each experimental group was calculated. In Group W, the infarction range was significantly reduced compared to the other two groups.

9C. The transcript amount of tumor necrosis factor alpha (TNF-$\alpha$) in the infarction area was quantified. In Group W, TNF-$\alpha$ transcript was significantly suppressed compared to Group C. No significant difference was observed in Group K compared to Group C.

9D. The number of CD11b positive cells at the boundary between the myocardial infarction site and the normal site was quantified in each group. In Groups W and K, infiltration of CD11b positive cells was reduced compared to Group C.

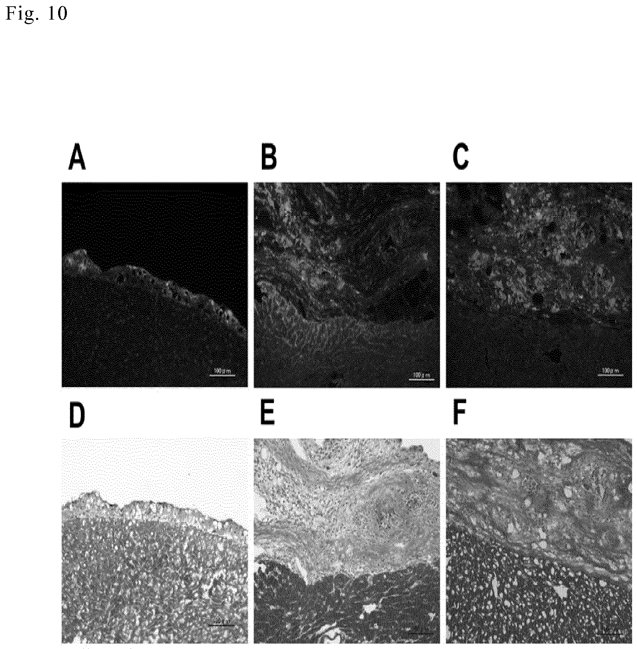

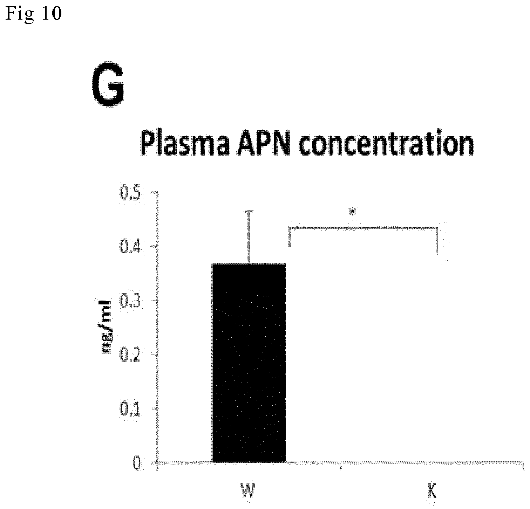

Fig. 10 shows adiponectin continuous production ability by the adipocyte sheets of Example 2.

10A. The result of a tissue in which a wild-type mouse-derived adipocyte sheet was implanted into the left ventricle of an adiponectin knockout mouse, and 1 hour after the implantation, the tissue was subjected to immunostaining with adiponectin. The light portion in the figure shows adiponectin.

10B. The result of a tissue in which a wild-type mouse-derived adipocyte sheet previously labeled with a red fluorescent dye was implanted into the left ventricle of an adiponectin knockout mouse, and 4 weeks after the implantation, the tissue was subjected to immunostaining with adiponectin. The cell sheet engrafted into the cardiac surface. Adiponectin was expressed in the adipocyte sheet and around cardiac muscle that is in contact with the adipocyte sheet. The light portion in the upper-left of the figure indicates the adipocyte sheet, and the light portion below the adipocyte sheet indicates adiponectin.

10C. The result of a tissue in which an adiponectin knockout mouse-derived adipocyte sheet previously labeled with a red fluorescent dye was implanted into the left ventricle of an adiponectin knockout mouse, and 4 weeks after the implantation, the tissue was subjected to immunostaining with adiponectin. The cell sheet engrafted into the cardiac surface. Adiponectin was not expressed in the adipocyte sheet and around cardiac muscle that is in contact with the adipocyte sheet. The light portion in the upper-left of the figure indicates the adipocyte sheet, and the light portion below the adipocyte sheet indicates adiponectin. 10D. The continuous section of Fig. 10A was subjected to hematoxylin eosin staining. The cell sheet was formed of adipocytes in which cytoplasmic fat droplets were accumulated as in the cell sheet *in vitro;* and the thickness was nearly the same, i.e., about 100 $\mu$m.

10E. The continuous section of Fig. 10B was subjected to hematoxylin eosin staining. The thickness of the cell sheet was increased, i.e., to about 600 $\mu$m, compared to the thickness one hour after the implantation. The cell sheet included connective tissue and granulation in addition to adipocytes in which cytoplasmic lipid droplets were accumulated.

10F. The continuous section of Fig. 10C was subjected to hematoxylin eosin staining. The adiponectin knockout mouse-derived sheet was in the same form as the wild-type mouse-derived adipocyte sheet 4 weeks after implantation, and had a thickness of about 600 $\mu$m. The cell sheet included connective tissue and granulation in addition to adipocytes in which cytoplasmic lipid droplets were accumulated.

10G. An effect on the blood adiponectin level by adipocyte sheet implantation is shown. A wild-type mouse-derived sheet and an adiponectin knockout mouse-derived sheet were implanted to the all left ventricular wall of the adiponectin knockout mouse. 4 weeks after the implantation, the plasma adiponectin amount was measured. In the adiponectin knockout mouse-derived adipocyte sheet implantation group (K), adiponectin was lower than the detection limit; however, in the wild-type mouse-derived adipocyte sheet implantation group (W), $0.37\pm0.098$ ng/ml adiponectin was detected.

Mode for Carrying out the Invention

Adiponectin is a cytokine secreted by adipose tissue. Adiponectin contributes to the control of lifestyle diseases and also has other effects, such as anti-apoptosis, anti-fibrosis, proangiogenesis, myocardial hypertrophy inhibition, and cell growth promotion. Further, adiponectin is effective for cardiovascular diseases, such as heart failure and arteriosclerosis. In the transplantation of adipocytes for left ventricle myocardial infarction, the secretion of adiponectin at the injured site alleviates the injury earlier and ameliorates inflammatory responses. Further, post-transplant middle- or long-term secretion of adiponectin by adipocytes grafted onto the affected site suppresses remodeling, such as thinning of the anterior left ventricular wall and fibrosis of the periphery of the infarcted area to thereby promote functional recovery. In the present invention, a drug delivery system (DDS) is constructed using adipocytes that are a biological material. According to the present invention, there are no biocompatibility concerns, and the graft material itself is grafted onto the injured site. Therefore, long-term local provision of highly active adiponectin is expected. Conventional cell sheets for treating heart failure are produced by using tissue stem cells, such as skeletal muscle myoblasts and bone marrow-derived mesenchymal stem cells. Compared to these cells, adipocytes are easily obtained and minimally invasive and can be produced in a large amount. Therefore, adipocytes are expected to be more widely applicable to various patients.

The present invention provides a cell sheet that comprises at least adipocytes and that is detached from a cell culture support for treating heart disease; and a three-dimensional cell sheet structure thereof. The present invention was accomplished based on the finding that the cell sheet and three-dimensional structure thereof comprising adipocytes are highly useful for treating heart failure after myocardial infarction. The adipocytes as used herein refer to cells differentiated into adipocytes. The type of adipocyte is not limited. For example, the cells may be mature adipocytes. The source of the adipocytes is not particularly limited. Examples of sources include adipose tissue, subcutaneous adipose tissue, epicardial adipose tissue, and the like. The method for obtaining adipocytes from such tissue is not particularly limited. Examples of usable methods include a method of directly harvesting adipocytes from easily available adipose tissue; a method comprising harvesting adipocytes, adipose stem cells, fibroblasts, or the like obtained from adipose tissue and inducing differentiation according to a usual method; etc. The method for separating adipocytes from such adipose tissue is not particularly limited. Examples of usable methods include the ceiling culture method, methods using fresh adipose tissue as is; etc. Examples of cells used as adipocytes in the present invention include mesenchymal stem cells that have grown in an undifferentiated state, and may also include other cells that are differentiated from mesenchymal stem cells, such as fibroblasts, interstitial cells, adipocytes, vascular endothelial cells, vascular endothelial progenitor cells, smooth muscle cells, SP cells, and myocardial cells as well as cells that enter during the process of harvesting mesenchymal stem cells, such as interstitial cells, fibroblasts, adipocytes, vascular endothelial cells, vascular endothelial progenitor cells, smooth muscle cells, SP cells, and myocardial cells. The cell sheet of the present invention preferably contains adipocytes in an amount of at least 50%, and more preferably 60% or more, 70% or more, 80% or more, 90% or more, or 95% or more. In the graft material for treating heart disease comprising cell sheets of the present invention, at least one of the cell sheets preferably contains adipocytes in an amount of at least 50%, and more preferably 60% or more, 70% or more, 80% or more, 90% or more, or 95% or more. In the graft material for treating heart disease comprising cell sheets of the present invention, adipocytes preferably account for at least 50%, and more preferably 60% or more, 70% or more, 80% or more, 90% or more, or 95% or more of the all the cells contained in the cell sheets. Further, in the graft material for treating heart disease comprising cell sheets of the present invention, all of the cell sheets preferably contain adipocytes in an amount of at least 50%, and more preferably 60% or more, 70% or more, 80% or more, 90% or more, or 95% or more.

According to the present invention, either myoblasts or mesenchymal stem cells, or both may be used in combination with adipocytes. The myoblasts are not particularly limited in regard to type and source tissue. For example, skeletal myoblasts of skeletal muscle tissue are preferable due to their abundant presence in the body and availability through a relatively easy operation. Furthermore, since the prior art has suggested that skeletal myoblasts can be transplanted into a heart, skeletal myoblasts can be used in actual medical practice. In the present invention, when myoblasts are harvested, the obtained cells may include, in addition to mesenchymal stem cells that have grown in an undifferentiated state, other cells that are differentiated from mesenchymal stem cells, such as fibroblasts, interstitial cells, adipocytes, vascular endothelical cells, vascular endothelial progenitor cells, and smooth muscle cells as well as cells that enter during the process of harvesting mesenchymal stem cells, such as interstitial cells, fibroblasts, adipocytes, vascular endothelial cells, vascular endothelial progenitor cells, smooth muscle cells, SP cells, and myocardial cells. In the present invention, the source of mesenchymal stem cells is not particularly limited. Easily available bone marrow or adipose tissue is preferable. As the cell source, somatic stem cells that have self-renewal properties and that are capable of differentiating into myocardial cells and vascular endothelial cells are preferable. However, the cell source is not particularly limited. Furthermore, since the prior art has suggested that mesenchymal stem cells can be transplanted into the heart, skeletal myoblasts can be used in actual medical practice. The patient's autologous somatic stem cells are particularly preferable from the viewpoint of histocompatibility and the risk of infection in transplantation. However, there is no particular limitation thereto. The mesenchymal stem cells may include, in addition to mesenchymal stem cells that have grown in an undifferentiated state, other cells that are differentiated from mesenchymal stem cells, such as fibroblasts, interstitial cells, adipocytes, vascular endothelical cells, vascular endothelial progenitor cells, smooth muscle cells, SP cells, and myocardial cells as well as cells that enter during the process of harvesting mesenchymal stem cells, such as interstitial cells, fibroblasts, adipocytes, vascular endothelial cells, vascular endothelial progenitor cells, smooth muscle cells, SP cells, and myocardial cells. The method of using these cells in combination is not particularly limited. Examples of usable methods include a method of co-culturing cells on the same culture surface, a method of culturing different types of cells via the surface of a porous culture insert such as a cell insert, a method of layering different types of cell sheets as described below, and a combination of these methods.

The present invention is achieved based on the finding that adiponectin produced by adipocytes has an anti-apoptotic effect and a growth-promoting effect on neighboring cells. The neighboring cells include, in addition to the cells of injured tissue, other cells that are present on the adipocyte sheet. In the present invention, it is necessary to use at least adipocytes. Other cells may also be present in myoblasts and mesenchymal stem cells. The cells are not particularly limited.

In the present invention, it is necessary to culture adipocytes. The medium used for the culture may be any general medium in which adipocytes can be cultured and is not particularly limited. Specific examples thereof include culture media supplemented with 10 to 15% autoserum or fetal bovine serum (FBS), 200 $\mu$M ascorbic acid, 0.5 to 2.0 $\mu$M insulin, and an antibiotic, such as $\alpha$-MEM, DMEM, F-12 medium, or a mixture thereof. A growth factor, such as fibroblast growth factor (bFGF) or adrenomedullin, may be added as needed. The cells may be cultured under any conditions suitable for mammalian cell cultures. In general, the cells are cultured under the conditions of 5% $CO_2$ at 37°C. The culture medium may be replaced as needed.

In the present invention, the cells including thus cultured adipocytes are harvested in the form of a sheet and the sheet is used to regenerate myocardial tissue. The method of harvesting the cultured cells in the form of a sheet is not particularly limited. Examples of usable methods include a method of culturing on a temperature-responsive cell culture substrate as described below, a method using a dilute aqueous solution of protease, a method using a specific aqueous solution of protease, a method of detaching the cells using aqueous EDTA alone, and a method of physically detaching the cells using a scraper or the like. In the present invention, the cell sheets thus obtained may be superposed and laminated to form a three-dimensional structure. Each cell sheet may contain both myoblasts and mesenchymal stem cells, or may consist of either myoblasts or mesenchymal stem cells and be superposed for co-culturing. The state of each sheet is not particularly limited. In the present invention, the shrinkage level of the cell sheets upon lamination of the cell sheets is not particularly limited, and other lamination conditions are also not particularly limited. Although the number of laminated sheets is not particularly limited, the number of laminations is preferably not more than 10, more preferably 8 or less, and still more preferably 4 or less. Lamination of the cell sheets is preferable because it increases the cell density per sheet unit area and enhances cell sheet functions. When a three-dimensional structure thereof is produced, other scaffolds, such as a gel or matrigel, which comprise at least one member selected from collagen, fibrin, gelatin, and the like, may be used. Although it is not restrictive in the present invention, it is preferable not to use such a material because the material may exert various effects in vivo after implantation.

In the present invention, the thickness of the adipocyte sheet peeled off from the surface of a culture substrate or a three-dimensional structure thereof is 50 $\mu$m or more. The thickness of the adipocyte sheet or three-dimensional structure thereof is preferably 50 $\mu$m or more, more preferably 70 $\mu$m or more, still more preferably 80 $\mu$m or more, and most preferably 100 $\mu$m or more. If the thickness of the adipocyte sheet or three-dimensional structure thereof is 50 $\mu$m or less, handling of the cell sheet becomes more difficult, which is not preferable in the present invention.

The adipocyte sheet and three-dimensional structure thereof according to the present invention must have an adiponectin secretory capacity of $3\times10^{-14}$ g/cell or more per day. The adipocyte sheet and three-dimensional structure thereof according to the present invention preferably have an adiponectin secretory capacity of $9\times10^{-14}$ g/cell or more per day, more preferably $1.2\times10^{-13}$ g/cell or more per day, still more preferably $1.6\times10^{-13}$ g/cell or more per day, and most preferably $2\times10^{-13}$ g/cell or more per day. If the adiponectin secretory capacity is $3\times10^{-14}$ g/cell per day or less, the implanted cell sheet will not be fully functional for regenerating myocardial tissue, which is undesirable.

Cells that can be used in the present invention include cells directly obtained from biological tissue, cells directly obtained from biological tissue and differentiated in a culture system, and cell lines. The type of cell or cell line is not particularly limited. The source of the cells is also not particularly limited. Examples of sources include, but are not limited to, humans, rats, mice, guinea pigs, marmosets, rabbits, dogs, cats, sheep, pigs, chimpanzees, and immunodeficient animals thereof. When the cell sheet or three-dimensional structure thereof according to the present invention is used to treat humans, cells derived from humans, pigs, or chimpanzees are preferably used. The medium used for cell culturing in the present invention is not particularly limited as long as the medium is the one usually used for the cells to be cultured.

In the present invention, the number of cells seeded in culturing varies depending on the animal species of the cells used. In general, the number of cells is preferably 3,000 to 20,000 per cm$^2$, more preferably 5,000 to 15,000 per cm$^2$, and still more preferably 6,000 to 10,000 per cm$^2$. A seeding concentration of 3,000 or more per cm$^2$ results in low adipocyte differentiation frequency and low secretion of adiponectin from the obtained cell sheet, which is not preferable in the present invention. The culture period until differentiation induction after seeding varies depending on the animal species of the cells used. In general, the culture period is preferably 5 to 10 days, and more preferably 7 to 8 days. A shorter culture period than this results in a low adipocyte differentiation frequency and low secretion of adiponectin from the obtained cell sheet, which is not preferable in the present invention. In the present invention, it is necessary to culture mature adipocytes. The culture medium may be any general medium in which mature adipocytes can be cultured and is not particularly limited. Specific examples thereof include culture media supplemented with 10 to 15% autoserum or fetal bovine serum (FBS), 0.5 to 2.0 $\mu$M insulin, 0.1 to 1.0 $\mu$M dexamethasone, and 0.2 to 2 $\mu$M isobutylmethylxanthine, such as $\alpha$-MEM, DMEM, F-12 medium, or a mixture thereof. Preferably, 5 $\mu$M pioglitazone, 200 $\mu$M ascorbic acid, and antibiotics are added to the medium, and growth factors such as a fibroblast growth factor (bFGF) and adrenomedullin may be added as needed. Generally, the differentiation induction period is preferably 36 to 60 hours, and more preferably 45 to 51 hours. A differentiation induction period shorter than this results in a low adipocyte differentiation frequency, whereas a differentiation induction period longer than this causes cell death, neither of which is preferable in the present invention. Generally, the culture period until implantation after differentiation induction is preferably 5 to 14 days, and more preferably 7 to 10 days. A culture period shorter than this results in a low level of maturation for the adipocytes and a smaller amount of adiponectin secreted, which are not preferable in the present invention.

The thus obtained cell sheet and three-dimensional structure thereof exhibit excellent anti-inflammatory effects, fibrosis inhibitory effects, angiogenic effects, and apoptosis inhibitory effects, and are advantageously used for regenerating myocardial tissue. The cell sheet and three-dimensional structure thereof according to the present invention express a hepatocyte growth factor (HGF) and a vascular endothelial cell growth factor (VEGF), which are useful for regenerating myocardial tissue. It was also revealed that these effects and the amounts of HGF and VEGF produced are remarkably increased, compared to those achieved by the presence of myoblasts or mesenchymal stem cells alone.

In the present invention, the cell sheet and three-dimensional structure thereof can be easily obtained by culturing on a cell culture support having a surface coated with a polymer whose hydration force changes in the temperature range of 0 to 80°C. More specifically, culturing is performed on a cell culture support having a surface coated with a polymer whose hydration force changes in the temperature range of 0 to 80°C, in a temperature range in which the polymer has weak hydration force. Generally, the temperature is preferably 37°C, which is a temperature at which cells are cultured. The temperature-responsive polymer used in the present invention may be a homopolymer or a copolymer. Examples of such a polymer include the polymer described in Japanese Unexamined Patent Publication No. H02-211865. More specifically, the temperature-responsive polymer can be obtained, for example, by homo- or co-polymerization of the following monomers. Examples of usable monomers include (meth)acrylamide compounds, N- (or N, N-di)alkyl-substituted (meth)acrylamide derivatives, or vinyl ether derivatives. The copolymer may comprise any two or more of these monomers. It is also possible to use a copolymer comprising monomers other than the above-described monomers, a copolymer obtained by graft- or co-polymerization of polymers, or a mixture of a polymer and a copolymer. Such a polymer may be cross-linked to such an extent that the inherent properties of the polymer are not impaired. Because the cells are cultured and peeled off in the process and the separation is performed in the temperature range of 5°C to 50°C, examples of usable temperature-responsive polymers include poly-N-n-propylacrylamide (lower critical solution temperature of the homopolymer: 21°C), poly-N-n-propylmethacrylamide (lower critical solution temperature of the homopolymer: 27°C), poly-N-isopropylacrylamide (lower critical solution temperature of the homopolymer: 32°C), poly-N-isopropylmethacrylamide (lower critical solution temperature of the homopolymer: 43°C), poly-N-cyclopropylacrylamide (lower critical solution temperature of the homopolymer: 45°C), poly-N-ethoxyethylacrylamide (lower critical solution temperature of the homopolymer: about 35°C), poly-N-ethoxyethylmethacrylamide (lower critical solution temperature of the homopolymer: about 45°C), poly-N-tetrahydrofurfurylacrylamide (lower critical solution temperature of the homopolymer: about 28°C), poly-N-tetrahydrofurfurylmethacrylamide (lower critical solution temperature of the homopolymer: about 35°C), poly-N,N-ethylmethylacrylamide (lower critical solution temperature of the homopolymer: 56°C), poly-N,N-diethylacrylamide (lower critical solution temperature of the homopolymer: 32°C), and the like. Examples of monomers used for copolymerization in the present invention include, but are not limited to, polyacrylamide, poly-N,N-diethylacrylamide, poly-N,N-dimethylacrylamide, polyethylene oxide, polyacrylic acid and salts thereof, polyhydroxyethyl methacrylate, polyhydroxyethyl acrylate, polyvinyl alcohol, polyvinyl pyrrolidone, cellulose, and carboxymethyl cellulose, and like aqueous polymers.

The method of coating the substrate surface with such a polymer according to the present invention is not particularly limited. For example, coating may be performed by subjecting the substrate and the above-mentioned monomers or polymers to any one of electron beam (EB) irradiation, $\gamma$-ray irradiation, ultraviolet irradiation, plasma treatment, corona treatment, and organic polymerization reaction or by means of physical adsorption as effected by application of a coating composition or kneading. The coating amount of the temperature-responsive polymer formed on the surface of the culture substrate is preferably in the range of 1.1 to 2.3 $\mu g/cm^2$, more preferably 1.4 to 1.9 $\mu g/cm^2$, and still more preferably 1.5 to 1.8 $\mu g/cm^2$. A coating amount of less than 1.1 $\mu g/cm^2$ is not preferable because cells on the polymer do not easily detach from the polymer even under stimulation and operating efficiency is considerably lowered. On the other hand, if the coating amount is 2.3 $\mu g/cm^2$ or more, cells do not easily adhere to the coated area, and adequate adhesion of the cells becomes difficult to achieve. In this case, if the temperature-responsive polymer coating layer is further coated with a cell adhesion protein, the coating amount of the temperature-responsive polymer on the substrate surface may be 2.3 $\mu g/cm^2$ or more. In this case, the coating amount of the temperature-responsive polymer is preferably 9.0 $\mu g/cm^2$ or less, more preferably 8.0 $\mu g/cm^2$ or less, and particularly preferably 7.0 $\mu g/cm^2$ or less. A coating amount of the temperature-responsive polymer of 9.0 $\mu g/cm^2$ or more is not preferable because cell adhesion becomes difficult even when the temperature-responsive polymer coating layer is further coated with a cell adhesion protein. The type of cell adhesion protein for use is not particularly limited. Examples of cell adhesion proteins include collagen, laminin, laminin 5, fibronectin, Matrigel, and the like. Such cell adhesion proteins can be used singly or in a combination of two or more. The method of coating with such a cell adhesion protein may be a conventional one. A typical coating method comprises applying an aqueous solution of a cell adhesion protein to the substrate surface and then removing the solution and rinsing. The present invention is a technique intended to preferably make use of the cell sheet itself by utilizing a temperature-responsive culture dish. Accordingly, an excessively large coating amount of cell adhesion protein on the temperature-responsive polymer layer is not preferable. The measurement of the coating amount of the temperature-responsive polymer and the coating amount of the cell adhesion protein may be according to conventional methods. Examples of usable methods include a method of directly measuring the site of cell adhesion by FT-IR-ATR, and a method comprising immobilizing a labeled polymer on the site of cell adhesion by a similar method and estimating the coating amount from the amount of the immobilized labeled polymer. Any of such methods can be used.

To detach and harvest the cultured cell sheet from the temperature-responsive substrate by the method of the present invention, the temperature of the culture substrate having cultured cells adhering thereto is adjusted to be equal to or higher than the upper critical solution temperature of the polymer coating on the culture substrate or to be equal to or lower than the lower critical solution temperature thereof, thereby detaching the cell sheet. The sheet may be detached in the culture medium or in other isotonic solutions, depending on the purpose. In order to detach and harvest the cells more quickly and efficiently, a method in which the cell culture substrate is lightly tapped and shaken, a method in which the culture medium is stirred using a pipette, etc., may be used alone or in combination. Culture conditions other than temperature may be conventional and are not particularly limited. For example, the culture medium may be supplemented with known fetal calf serum (FCS) or like serum, or alternatively, serum-free medium without the supplementation of such a serum may be employed. Further, ascorbic acid or a derivative thereof may be added to the medium to allow the cells to sufficiently produce an extracellular matrix.

The above description is exemplified below by referring to poly(N-isopropylacrylamide) as an example of the temperature-responsive polymer. Poly(N-isopropylacrylamide) is known as a polymer having a lower critical solution temperature of 31°C. In a free state, poly(N-isopropylacrylamide) is dehydrated in water at a temperature of 31°C or higher and the polymer chains agglomerate to cause turbidity. Conversely, at a temperature of 31°C or lower, the polymer chains are hydrated and dissolve in water. In the present invention, a coating of this polymer is formed and immobilized on the surface of a substrate such as a Petri dish. Therefore, at a temperature of 31°C or higher, the polymer on the substrate surface is dehydrated likewise but with the polymer chains being immobilized to form a coating on the substrate surface, the substrate surface becomes hydrophobic. On the other hand, at a temperature of 31°C or lower, the polymer on the substrate surface is hydrated but with the coat of polymer chains being immobilized on the substrate surface, the substrate surface becomes hydrophilic. This hydrophobic surface is suitable for cell adhesion and cell growth, whereas the hydrophilic surface becomes a surface to which the cells cannot adhere, and the cells in culture or the cell sheet can be detached only by cooling.

Examples of the substrate to be coated include, but are not limited to, materials typically used in cell culturing, such as glass, modified glass, polystyrene, polymethylmethacrylate, polyethylene terephthalate, polycarbonate, and like compounds, and substantially all materials that can generally be shaped, such as ceramics and polymer compounds other than those mentioned above.

The shape of the culture substrate in the present invention is not particularly limited. For example, the culture substrate may be in the form of a dish, a multi-well plate, a flask, a cell insert, beads, a flat membrane, a porous membrane, etc.

The cell sheet and three-dimensional structure thereof according to the present invention are not damaged by proteolytic enzymes, such as dispase or trypsin during culturing. Therefore, the cell sheet and three-dimensional structure thereof detached from the substrate have an adherent protein. When the cell sheet or three-dimensional structure thereof is detached in the form of a sheet, the cell-to-cell desmosome structure is maintained to some extent. Therefore, the cell sheet and three-dimensional structure thereof can well adhere to the tissue of an affected part upon implantation, and implantation can be efficiently performed. It is generally known that when using a dispase that is a proteolytic enzyme, the cell-to-cell desmosome structure can be detached while maintaining 10 to 40% thereof. However, because a cell-to-substrate basement membrane-like protein or the like is mostly destroyed, the strength of the obtained cell sheet becomes weak. In contrast to this, the cell sheet and three-dimensional structure thereof according to the present invention is such that 60% or more of both the desmosome structure and the basement membrane-like protein remain, and various effects as described above can be obtained.

The method of constructing a three-dimensional structure in the present invention is not particularly limited. For example, the cultured cells are detached in the form of a sheet, and a plurality of cultured sheets thus obtained are piled up using a jig for transferring the cultured cells to form a three-dimensional structure. The temperature of the medium is not particularly limited insofar as the following conditions are met. When the polymer that covers the surface of the culture substrate has an upper limit critical solution temperature, the temperature of the medium is set to be equal to or lower than the upper limit critical solution temperature. When the polymer that covers the surface of the culture substrate has a lower limit critical solution temperature, the temperature of the medium is set to be equal to or higher than the lower limit critical solution temperature. Of course, a low temperature range in which cultured cells cannot grow and a high temperature range in which cultured cells are killed are inappropriate. Culture conditions other than temperature may be conventional and are not particularly limited. The cell types of the stratified cell sheets are not particularly limited. Examples of usable methods include a method of laminating adipocyte sheets, a method of laminating an adipocyte sheet and a myoblast sheet, a method of laminating cell sheets obtained by co-culturing adipocytes and myoblasts, or a combination of these lamination methods. When the stacking cells are xenogeneic, the order of lamination of the cells is not particularly limited. The medium to be used may be supplemented with known fetal calf serum (FCS) or the like, or alternatively, a serum-free medium without the supplementation of such a serum may be employed. Further, the jig for transferring cultured cells is not particularly limited insofar as it can capture the detached cell sheet. Examples of usable jigs include porous membranes; paper, rubber and like membranes and plates; sponges; etc. To facilitate the laminating operation, a jig comprising a handle to which a porous membrane, paper, rubber or like membrane or plate, a sponge, or the like is attached may be used.

In the present invention, a carrier may be used for adhering the cell sheet detached from the temperature-responsive cell culture substrate and the three-dimensional structure thereof. For example, a polymeric membrane or a structure molded from the polymeric membrane, a metallic jig, or the like can be used as a carrier for maintaining the shape of the cell sheet and three-dimensional structure thereof. For example, when a polymer is used as a carrier material, specific examples of the material include polyvinylidene difluoride (PVDF), polypropylene, polyethylene, cellulose and derivatives thereof, paper, chitin, chitosan, collagen, urethanes, and gelatin. The shape of the carrier is not particularly limited.

The cell sheet and three-dimensional structure thereof obtained in the present invention can be implanted into a predetermined site within the living body. The implant site may be any site of myocardial tissue and is not particularly limited. Alternatively, in particular, greater omentum is preferable because of the abundant presence of blood vessels and ease of implantation. The cell sheet and three-dimensional structure thereof implanted into the omentum may be implanted into myocardial tissue while blood vessels remain joined. The graft site may or may not be previously subjected to the induction of angiogenesis and is not particularly limited. Herein, the method of inducing angiogenesis is also not particularly limited. Examples of usable methods include a method of embedding FGF (fibroblast growth factor), which is a blood growth factor, in a microsphere and allowing the microsphere to act within the living body for 8 to 10 days while changing the composition, size, and range of injection of the microsphere; a method of cutting a polyethylene terephthalate mesh to any size to prepare a bag, placing a solution of FGF in a high concentration agarose solution in the bag and then eliminating the bag after 8 to 10 days to prepare an angiogenesis induced space; and the like.

When the cell sheet or three-dimensional structure thereof according to the present invention is used for humans, the implanted cell sheet and three-dimensional structure thereof can function in the living body for a long period of time. The expression level of the function can be controlled by either one or both of the size and shape of the detached cell sheet and three-dimensional structure thereof. The cell sheet and three-dimensional structure thereof can be used to treat various diseases associated with disease or disorder selected from the group consisting of heart failure, ischemic heart disease, myocardial infarction, cardiomyopathy, myocarditis, hypertrophic cardiomyopathy, dilated phase hypertrophic cardiomyopathy, and dilated cardiomyopathy. However, the use of the cell sheet and three-dimensional structure thereof is not particularly limited.

When the cell sheet and three-dimensional structure thereof according to the present invention are implanted into an animal, the animal can be used for drug evaluation. The expression level of the function can be controlled by the size and shape of the detached cell sheet and three-dimensional structure thereof. Examples of animals that can be used include, but are not limited to, rats, mice, guinea pigs, marmosets, rabbits, dogs, pigs, chimpanzees, and immunodeficient animals. Such implantation animals can be used, for example, for a cardiac function assessment system, etc., after being administered with a test substance. However, the use of the animals is not particularly limited.

Examples

Hereunder, the present invention is described in greater detail with reference to examples, which are by no means intended to limit the invention.

In the examples below, animals were treated in accordance with rules defined by Osaka University (Japan) and were cared for in the spirit of animal protection.

Example 1

Preparation of adipose tissue derived fibroblasts

Subcutaneous adipose tissues were extracted from both the inguinal areas of 3-week-old male LEW/Sea rats. The adipose tissues were finely minced with scissors and suspended in a 0.1% type II collagenase solution, and the suspension was shaken in a 37°C bath for 1 hour. The result was filtered through a 250-$\mu$m-mesh filter and centrifuged for 5 minutes at 1,800 rpm. The sediment was suspended in a medium (containing 10% fetal calf serum, 200 $\mu$M of ascorbic acid, and antibiotic-containing D-MEM). The suspension was filtered through a 25-$\mu$m-mesh filter and centrifuged for 5 minutes at 1,800 rpm. The sediment was suspended in the medium, plated onto a culture dish, and cultured under a wet environment (5% carbon dioxide, 37°C). The cells that adhered to the culture dish for 24 hours following the initiation of culturing were determined to be adipose tissue derived fibroblasts (Stromal-Vascular Fraction cells: SVF cells).

Induction of differentiation into adipocytes and preparation of adipocyte sheet

Three days after the initiation of culturing, the cells were formed into a cell-suspended liquid by treatment with trypsin and plated on a 35-mm-diameter temperature-responsive culture dish at a cell density of 7,000 cells/cm$^2$. Ten days after the initiation of culturing, a differentiation-inducing medium (containing 10% fetal calf serum, 200 $\mu$M of ascorbic acid, 0.87 $\mu$M of insulin, 0.25 $\mu$M of dexamethasone, 500 $\mu$M of isobutylmethylxanthine, 5 $\mu$M of pioglitazone, and antibiotic-containing D-MEM) was added to the adipose derived fibroblasts that became confluent on the temperature-responsive culture dish for 48 hours to differentiate them into adipocytes. After the induction of differentiation was completed, the adipocytes were cultured in a maintenance medium (containing 10% fetal calf serum, 200 $\mu$M of ascorbic acid, 0.87 $\mu$M of insulin, and antibiotic-containing D-MEM) and cytoplasmic lipid droplets were accumulated, thereby confirming differentiation into adipocytes. After 19 to 25 days from the initiation of culturing, the adipocytes were cultured in a temperature-responsive culture dish at 20°C. Within 40 minutes, the adipocytes automatically peeled off and floated in the culture fluid as an adipocyte sheet.

Secretion of adiponectin in the adipocyte sheet

The medium, in which the adipocyte sheet was cultured for 24 hours, was collected and the amount of adiponectin in the culture fluid was measured by the enzyme linked immunosorbent assay (ELISA) method.

Histological analysis of adipocyte sheet

A frozen section of the adipocyte sheet was prepared and fixed using 4% paraformaldehyde. Thereafter, oil red O staining and adiponectin immunostaining were performed.

Cardiac failure model and implantation of adipocyte sheet

8-week-old female LEW/Sea rats were used. A myocardial infarction model was prepared by left anterior descending coronary artery ligation. After performing general anesthesia by means of isoflurane inhalation anesthesia under artificial respiration (1.5% isoflurane, ventilation amount: 4 ml, 100 cycles/min), a left thoracotomy was performed to expose the heart, and suturing with an 8-0 proline was made at a site 2 to 3 mm from the origin of the left coronary artery.

Fifteen minutes after the coronary artery ligation, the adipocyte sheet was gently washed with PBS, placed on rounded-end tweezers to slide it to the site of infarction, and then left to stand. Twenty minutes after the implantation, the chest was closed. The same procedure was performed on the untreated group but without implantation.

Animal experiment protocol

Fifteen minutes after the coronary artery ligation, the rats were divided into two groups and subjected to the following treatment. In one group, two adipocyte sheets were implanted in the left ventricular anterior wall (Group A: n = 15), and in the other group, the untreated group, implantation was not conducted (Group C: n = 15). Three days after the surgery, histological evaluation and molecular biological evaluation were conducted. Four weeks after the surgery, cardiac ultrasound test, cardiac catheter test, and histological evaluation were conducted.

Cardiac ultrasound test

After obtaining a suppression state by isoflurane inhalation anesthesia, a short-axis view of the left ventricle at the papillary-muscle level was obtained by using a cardiac ultrasound system with a 14-MHz transducer while monitoring the end-systolic blood pressure and cardiac rate. The left ventricular end-diastolic area (LVEDA) and left ventricular end-systolic area (LVESA) were measured. The measurement was repeated three times and the average value was obtained. The fractional area change (FAC) in the left ventricle was calculated by the following formula:

$$FAC\ (\%) = (LVEDA-LVESA)/LVEDA \times 100$$

Cardiac catheter test

After performing general anesthesia by means of isoflurane inhalation anesthesia under artificial respiration (1.5% isoflurane, ventilation amount: 4 ml, 100 cycles/min), a median thoracotomy was performed to expose the heart. The inferior vena cava was sutured with a 1-0 silk suture. A conductance catheter and a pressure catheter were inserted into the left ventricular cavity from the apex of the left ventricle and the left ventricular anterior wall, respectively. After extracting the PV-loop, the inferior vena cava was ligated to apply a load thereto. The data were analyzed using software (Integral 3, produced by Unique Medical Co., Ltd.).

Histological evaluation

Three days or twenty-eight days after the surgery, the heart was stopped in diastole by potassium administration and then excised. The left ventricle was sliced along the direction of the short axis, embedded in a compound, and frozen in liquid nitrogen to prepare tissue sections. The tissue sections underwent hematoxylin and eosin staining, Masson's trichrome staining, oil red O staining, adiponectin immunostaining, CD11b immunostaining, and TUNEL staining. The fibrosis ratio was obtained by analyzing the Masson's trichrome stain image. The infarct size was determined by an image analysis of the macro image of the tissue section stained by hematoxylin and eosin. Regarding CD11b immunostaining and TUNEL staining, the number of positive cells per visual field of view was counted and the average of five fields of view per specimen was calculated.

Molecular biological analysis

Three days after the surgery, the extracted cardiac sample was divided into the infarction site (scar), the boundary between infarction and normal (border), and the non-infarction site (normal), and each was subjected to RNA extraction. DNA was synthesized from RNA by a reverse transcription reaction. The transcript amounts of tumor necrosis factor (TNF-$\alpha$) and monocyte chemotactic and activating factor (MCP-1) were quantified by the quantitative PCR method. The transcript amounts thus obtained were divided by the transcript amount of glyceraldehyde-3-phosphate dehydrogenase (GAPDH), which is an endogenous control, and expressed by the ratio with the value of the normal group for each specimen.

Statistical analysis

The results were expressed as mean $\pm$ standard error. The comparison between groups was performed by a t-test. When the P value was less than 0.05, it was determined that there was a significant difference. The survival ratio analysis was conducted by the Kaplan Mayer method to calculate the survival ratio, and significant differences in each experimental group were obtained by a log-rank test.

Preparation of adipocyte sheet

Rat adipose tissue derived fibroblasts were induced to differentiate into adipocytes. Seven days from the initiation of differentiation induction, many oil red O-positive adipocytes were expressed (Fig. 1A). Adipocytes cultured on a 35-mm-diameter temperature-responsive culture dish were incubated at 20°C to form about a 10-mm-square sheet (Fig. 1B). The cell count per sheet was about $2 \times 10^6$. The adipocyte sheet had a thickness of about 100 $\mu$m and contained many oil red O-positive adipocytes, and adiponectin expression was observed in cytoplasm (Figs. 1C and 1D). The secretion of $0.98 \pm 0.20$ $\mu$g/ml of adiponectin was observed in the culture fluid in which the adipocyte sheet was cultured for 24 hours. In contrast, the cell culture fluid itself and a culture fluid for an adipose derived fibroblast sheet without a differentiation-induction treatment exhibited a concentration of adiponectin lower than the detection limit.

Grafting of adipocyte sheet at myocardial infarction site

The adipocyte sheet implanted into the left ventricular anterior wall during the acute myocardial infarction was well grafted to the scar area of the left ventricular anterior wall 28 days after the implantation (Fig. 1E). The adipocyte sheet was oil red O-positive and adiponectin positive even at the graft site (Figs. 1F to 1H).

Effect of adipocyte sheet on cardiac function

Figs. 2A to 2G show the evaluation of cardiac function of the 28 days after implanting the sheet in the acute myocardial infarction. The cardiac ultrasound test results showed that the values of LVEDA and LVESA in Group A were smaller than those in Group C, and this indicates that enlargement of the left ventricle was suppressed (Figs. 2A and 2B). The FAC in Group A was significantly larger than that in Group C. This indicates that the deterioration of cardiac function was suppressed by the implantation of the sheet (Fig. 2C). In the cardiac catheter test, the LV maximum positivity dP/dt (dP/dtmax), which is an index used to indicate contractility and LV end-systolic elastance (Ees), increased, and the LV maximum negative dP/dt (dP/dtmin), which is an index used to indicate expansibility and LV isovolumic relaxation time ($\tau$), decreased. This indicates that the implantation of the adipocyte sheet provides a therapeutic effect in treating acute myocardial infarction.

Effect of adipocyte sheet on myocardial tissue

Twenty-eight days after the sheet implantation in response to acute myocardial infarction, a histological analysis was conducted. A remarkable thinning of the left ventricular anterior wall was observed in Group C (the untreated group). In contrast, the left ventricular anterior wall was thicker in Group A compared to that in Group C. This indicates that the tissues of the left ventricular anterior wall were maintained (Figs. 3A to 3C). Further, the formation of fibrous tissue (fibrosis) in the periphery of the infarction site was evaluated by employing Masson's trichrome staining. The results showed that Group A exhibited significant suppression of the fibrosis compared to Group C (Figs. 3D to 3F).

Myocardial protection effect of adipocyte sheet

Three days after the implantation of the adipocyte sheet in response to acute myocardial infarction, a histological analysis was conducted. Each of the infarct sizes was quantitated. The results showed that the infarct size in Group A was significantly smaller than that in Group C (Figs. 4A to 4C). The apoptotic cells in the periphery of the myocardial infarction site were detected by TUNEL staining, and the results revealed that the number of TUNEL positive cells in Group A was significantly smaller than that in Group C (Figs. 4D to 4F). These analytical results indicate that infarct size was limited by the implantation of the adipocyte sheet through suppression of apoptosis.

Anti-inflammatory effect of adipocyte sheet

Fig. 5 shows an analysis of the anti-inflammatory effect of an adipocyte sheet on myocardial tissue after 3 days from the implantation. The infiltration of inflammatory cells into the periphery of the myocardial infarction was evaluated by CD11b immunostaining, which is an inflammatory cell marker. The number of CD11b positive cells in the periphery of the infarction site of Group A was significantly lower than that of Group C (Figs. 5A to 5C). Furthermore, the degree of transcription of inflammatory cytokine in the infarction site and that in the periphery of the infarction site were evaluated. The results revealed that Group A exhibited significantly lower transcript amounts of TNF-$\alpha$ and MCP-1 both in the infarction site and in the periphery of the infarction site compared with those of Group C (Figs. 5D and 5E). The above-described results indicate that the inflammatory reaction, including the infiltration of inflammatory cells after the myocardial infarction, was likely to have been alleviated by the implantation of the adipocyte sheet.

Example 2

Preparation of adipose tissue derived fibroblasts

Subcutaneous adipose tissues were extracted from both the inguinal areas of 20-week-old male C57BL/6J (wild-type) mice and genetically modified (adiponectin knockout) mice. The adipose tissues were finely minced with scissors and suspended in a 0.1% type II collagenase solution, and the suspension was shaken in a 37°C bath for 1 hour. The result was filtered through a 100-$\mu$m-mesh filter and centrifuged for 5 minutes at 1,800 rpm. The sediment was suspended in a medium (containing 10% fetal calf serum, 200 $\mu$M of ascorbic acid, and antibiotic-containing D-MEM). The suspension was filtered through a 70-$\mu$m-mesh filter and centrifuged for 5 minutes at 1,800 rpm. The sediment was suspended in the medium, plated onto a culture dish, and cultured under a wet environment (5% carbon dioxide, 37°C). The cells that adhered to the culture dish for 24 hours following the initiation of culturing were determined to be adipose derived fibroblasts (Stromal-Vascular Fraction cells: SVF cells).

Induction of differentiation into adipocyte and preparation of adipocyte sheet

Three days after the initiation of culturing, the cells were formed into a cell-suspended liquid by treatment with trypsin and plated on a 24-hole (diameter: 15.5 mm) temperature-responsive culture dish at a cell density of 7,000 cells/cm2. Ten days after the initiation of culturing, a differentiation-inducing medium (containing 10% fetal calf serum, 200 $\mu$M of ascorbic acid, 0.87 $\mu$M of insulin, 0.25 $\mu$M of dexamethasone, 500 $\mu$M of isobutylmethylxanthine, 5 $\mu$M of pioglitazone, and antibiotic-containing D-MEM) was added to the adipose derived fibroblasts that became confluent on the temperature-responsive culture dish for 48 hours to induce them to differentiate into adipocytes. After the induction of differentiation was completed, the cells were cultured in a maintenance medium (containing 10% fetal calf serum, 200 $\mu$M of ascorbic acid, 0.87 $\mu$M of insulin, and antibiotic-containing D-MEM) and cytoplasmic lipid droplets were accumulated, thereby confirming differentiation into adipocytes. After 19 to 25 days from the initiation of culturing, the cells were cultured in a temperature-responsive culture dish at 20°C. The adipocytes automatically peeled off within 40 minutes and floated in the culture fluid as an adipocyte sheet.

Secretion of adiponectin in adipocyte sheet

The medium, in which the adipocyte sheet was cultured for 24 hours, was collected and the amount of adiponectin in the culture fluid was measured by an enzyme linked immunosorbent assay (ELISA) method.

Histological analysis of adipocyte sheet

Frozen sections of the adipocyte sheet were prepared and fixed with 4% paraformaldehyde. Thereafter, hematoxylin and eosin staining and adiponectin immunostaining were performed.

Cardiac failure model and implantation of adipocyte sheet

Male C57BL/6J (wild-type) mice and genetically modified (adiponectin knockout) mice were used. A myocardial infarction model was prepared by ligation of the left anterior descending coronary artery. After performing general anesthesia by means of isoflurane inhalation anesthesia under artificial respiration (1.0% isoflurane, ventilation amount: 1 ml, 100 cycles/min), a left thoracotomy was performed to expose the heart, and an 8-0 proline suture was made at a site about 1 mm from the origin of the left coronary artery.

Fifteen minutes after the coronary artery ligation, the adipocyte sheet was gently washed with PBS, placed on rounded-end tweezers to slide it to the site of infarction, and then left to stand. Twenty minutes after the implantation, the chest was closed. The same procedure was performed on the untreated group but without implantation.

Animal experiment protocol

Fifteen minutes after the coronary artery ligation, the rats were divided into three groups and subjected to the following treatment. Specifically, two adipocyte sheets derived from wild-type mice were implanted in the left ventricular anterior wall (Group W), two adipocyte sheets derived from adiponectin knockout mice were implanted in the left ventricular anterior wall (Group K), and no implantation was conducted for the untreated group (Group C). Two days after the surgery, histological evaluation and molecular biological evaluation were conducted. Four weeks after the surgery, cardiac ultrasound test and histological evaluation were conducted.

Cardiac ultrasound test

After obtaining a suppression state by isoflurane inhalation anesthesia, a short-axis view of the left ventricle at the papillary-muscle level was obtained by using a cardiac ultrasound system with a 14-MHz transducer. The left ventricle dimension in diastole (LVDd) and the left ventricle dimension in systole (LVDs) were measured. The measurement was repeated three times and the average value was obtained. A left ventricle ejection fraction (EF) was calculated by the following formula: EF (%) = $(LVDd^3 - LVDs^3)/LVDd^3 \times 100$.

Histological evaluation

Two days or twenty-eight days after the surgery, the heart was stopped in diastole by potassium administration and then excised. The left ventricle was sliced along the direction of the short axis. The specimens 2 days after the implantation were subjected to TTC staining by a conventional method, embedded in a compound, and frozen in liquid nitrogen to prepare tissue sections. The tissue sections underwent hematoxylin and eosin staining, Masson's trichrome staining, PAS staining, oil red O staining, adiponectin immunostaining, and CD11b immunostaining. The fibrosis ratio was obtained by analyzing the Masson's trichrome stain image. The diameter of the cardiomyocyte was determined by analyzing the diameters of the images of PAS stained cardiomyocytes. The infarction range was obtained by analyzing TTC stained images. Regarding CD11b immunostaining, the number of positive cells per visual field of view was counted. In terms of the quantitative evaluations described above, an average of five fields of view per specimen was calculated, and the average value of each group was obtained.

Molecular biological analysis

Two days after the surgery, RNA was extracted from the infarction site of the excised cardiac sample. cDNA was synthesized from RNA by a reverse transcription reaction. The transcript amount of tumor necrosis factor (TNF-$\alpha$) was quantified by the quantitative PCR method. The transcript amount thus obtained was divided by the transcript amount of glyceraldehyde-3-phosphate dehydrogenase (GAPDH), which is an endogenous control.

Preparation of adipocyte sheet

Rat adipose tissue derived fibroblasts were induced to differentiate into adipocytes. Seven days from the initiation of the inducing differentiation, many adipocytes containing cytoplasmic lipid droplets accumulated therein were expressed (Fig. 6A). Adipocytes cultured on a 15.5-mm-diameter temperature-responsive culture dish were incubated at 20°C to form a sheet having a thickness of about 7 mm (Fig. 6B). The adipocyte sheet mainly comprised adipocytes containing cytoplasmic lipid droplets accumulated therein, and had a thickness of about 100 μm (Fig. 6C). In the adipocyte sheet derived from wild-type mice, the expression of adiponectin in the cytoplasm was observed. However, no expression of adiponectin was observed in the wild-type-mouse derived adipose derived fibroblast sheet without differentiation-induction treatment, adiponectin knockout-mouse derived adipocyte sheet, and adipose derived fibroblast sheet (Fig. 6D). Secretion of adiponectin was observed in the culture fluid in which the adipocyte sheet was cultured for 24 hours. However, the cell culture fluid itself and a culture fluid for a wild-type-mouse derived adipose derived fibroblast sheet and an adiponectin knockout-mouse derived adipocyte sheet exhibited a concentration of adiponectin lower than the detection limit.

Effect of adipocyte-sheet derived adiponectin on cardiac function

Figs. 7A and 7B show an evaluation of cardiac function 28 days after implanting the sheet in response to acute myocardial infarction. The results of cardiac ultrasound test showed that the LVDs values of Group W were significantly smaller than those of Group C. This indicates that the enlargement of the left ventricle was suppressed. The results of Group K showed a tendency toward smaller values both in LVDd and LVDs compared to Group C but no significant differences were observed. Regarding EF, Group W exhibited significantly higher results compared to Group C, and Group K, but showed no significant difference from Group C (Fig. 7A). These results indicate that the deterioration of cardiac function was suppressed by implanting an adipocyte sheet, and adipocyte-sheet derived adiponectin plays important role in the mechanism of the suppression. The survival ratio of each group was evaluated. The results revealed that Group W had a significantly higher survival ratio (p = 0.0428) compared to Group C. Although not so significant compared to Group K, Group W exhibited a slight improvement in survival ratio (p = 0.0614). The results described above suggested that the prognosis of patients suffering from myocardial infarction could be improved through exogenous adiponectin by implanting an adipocyte sheet.

Effect of adipocyte-sheet derived adiponectin on myocardial tissue

Histological analysis was conducted 28 days after a sheet was implanted in response to acute myocardial infarction. Group W had a significantly smaller cardiomyocyte diameter compared with those of Group K and Group C. This indicates that myocardial hypertrophy after myocardial infarction was suppressed (Figs. 8A and 8B). The formation of fibrous tissue (fibrosis) in the periphery of the infarction site was evaluated by Masson's trichrome staining, and the results showed that in Group W, the fibrosis was significantly suppressed compared to Group K and Group C (Figs. 8C and 8D).

Myocardial protection effect of adipocyte-sheet derived adiponectin

Histological analysis was conducted 2 days after an adipocyte sheet was implanted in response to acute myocardial infarction. The infarct size was quantitated by TTC staining, and the results revealed that the infarct size in Group W was significantly smaller than those of Group K and Group C (Figs. 9A and 9B). The degree of transcription of inflammatory cytokine TNF-α in the infarction site was evaluated, and the results revealed that Group W had a significantly lower transcript amount compared to Group K and Group C (Fig. 9C). The infiltration of inflammatory cells into the periphery of the myocardial infarction was evaluated by CD11b immunostaining, which is an inflammatory cell marker. The number of CD11b positive cells in the periphery of the infarction sites of Group W and Group K was significantly lower than that of Group C (Fig. 9D). The above-described results indicate that the inflammatory reaction, including the infiltration of inflammatory cells after the myocardial infarction, was likely to have been alleviated by the implantation of the adipocyte sheet.

Continuous production of adiponectin by adipocyte sheet

In order to evaluate the continuous production of adipocyte-sheet derived adiponectin and the infiltration to the myocardial tissue at the implantation site, an adipocyte sheet was implanted using adiponectin knockout mice as recipients. A wild-type-mouse derived adipocyte sheet was grafted to the myocardial tissue for 1 month from the time of implantation, and adiponectin was secreted. In the extracellular matrices (ECM) of cardiomyocytes that were in contact with the adipocyte sheet, adiponectin expression was observed. This indicates that the adipocyte-sheet derived adiponectin was infiltrated in the cardiomyocytes in the vicinity (Fig. 10B). Such an adiponectin positive immunostaining image in proximate cardiomyocytes was not obtained when an adiponectin knockout-mouse derived adipocyte sheet was used. Therefore, the possibility that the immunostained image of Fig. 10B was a non-specific case is eliminated (Fig. 10C). Furthermore, adipocyte-sheet derived adiponectin was detected from plasma 1 month after the adipocyte sheet was implanted. The adiponectin in the plasma of the mice implanted with an adiponectin knockout-mouse derived adipocyte sheet had a concentration that was at or below the detection limit (Fig. 10G).

Industrial Applicability

An extremely decreased cardiac function can be improved by the use of the adipocyte sheet of the present invention. Therefore, a therapeutic effect achieved by the use of such a cell sheet or a three-dimensional structure thereof can be expected. Furthermore, adipocyte sheet implantation can increase the concentration of adiponectin in blood, enabling the possibility of therapeutic effects on various diseases, including lifestyle-related diseases.

**Claims**

1. A graft material for use in the treatment of a heart disease comprising a cell sheet containing differentiated adipocytes and detached from a cell culture support.

2. The graft material for use in the treatment of a heart disease according to claim 1, wherein the cell sheet has a thickness of 50 $\mu$m or more.

3. The graft material for use in the treatment of a heart disease according to claim 1 or 2, wherein the cell sheet has an adiponectin secretory capacity of $3 \times 10^{-14}$ g/cell or more per day.

4. The graft material for use in the treatment of a heart disease according to any one of claims 1 to 3, wherein the cell sheet has at least one function selected from the group consisting of fibroblast-inhibiting function, angiogenic function, apoptosis-inhibiting function, and anti-inflammatory function.

5. The graft material for use in the treatment of a heart disease according to any one of claims 1 to 4, wherein the adipocytes are obtained by inducing differentiation of adipose tissue derived fibroblasts.

6. The graft material for use in the treatment of a heart disease according to any one of claims 1 to 5, comprising multiple cell sheets in a laminated state.

7. The graft material for use in the treatment of a heart disease according to any one of claims 1 to 6, wherein at least one of the cell sheets further comprises at least one selected from the group consisting of myoblasts and mesenchymal stem cells.

8. The graft material for use in the treatment of a heart disease according to claim 7, wherein the myoblast is a skeletal myoblast.

9. The graft material for use in the treatment of a heart disease according to claim 7 or 8, wherein the mesenchymal stem cell is derived from adipose tissue.

10. The graft material for use in the treatment of a heart disease according to any one of claims 1 to 9, wherein the graft material is applied to an individual from which the adipocytes are derived.

11. The graft material for use in the treatment of a heart disease according to any one of claims 1 to 10, further comprising a cytokine or growth factor.

12. The graft material for use in the treatment of heart disease according to any one of claims 1 to 11, wherein the heart disease is at least one disease or disorder selected from the group consisting of cardiac failure, ischemic heart disease, myocardial infarction, cardiomyopathy, myocarditis, hypertrophic cardiomyopathy, dilated-phase hypertrophic cardiomyopathy, and dilated cardiomyopathy.

13. A method for producing a graft material for treating heart disease comprising a cell sheet containing adipocytes, without using a scaffold, the method comprising the steps of:

   a) culturing a cell group containing adipocytes on a cell culture support coated with a temperature-responsive polymer whose upper critical solution temperature or lower critical solution temperature in water is 0 to 80°C, in a culture solution;

b) bringing the temperature of the culture solution to the upper critical solution temperature or above or the lower critical solution temperature or below; and

c) peeling off the cell group as a cell sheet from the cell culture support.

14. The method according to claim 13, further comprising step d) of adding an ascorbic acid or a derivative thereof to the culture solution before step c).

15. The method according to claim 13 or 14, comprising no step of protease treatment.

16. The method according to any one of claims 13 to 15, wherein the temperature-responsive polymer is poly(N-isopropylacrylamide).

**Patentansprüche**

1. Transplantatmaterial zur Verwendung bei der Behandlung einer Herzerkrankung, umfassend eine Zellschicht, die differenzierte und von einem Zellkulturträger abgelöste Adipozyten enthält.

2. Transplantatmaterial zur Verwendung bei der Behandlung einer Herzerkrankung gemäß Anspruch 1, wobei die Zellschicht eine Dicke von 50 $\mu$m oder mehr hat.

3. Transplantatmaterial zur Verwendung bei der Behandlung einer Herzerkrankung gemäß Anspruch 1 oder 2, wobei die Zellschicht eine sekretorische Fähigkeit von Adiponectin von 3 x $10^{-14}$ g/Zellen oder mehr pro Tag hat.

4. Transplantatmaterial zur Verwendung bei der Behandlung einer Herzerkrankung gemäß einem der Ansprüche 1 bis 3, wobei die Zellschicht mindestens eine Funktion hat, die ausgewählt ist aus der Gruppe bestehend aus Fibroblasten-hemmender Funktion, angiogener Funktion, Apoptosehemmender Funktion und anti-inflammatorischer Funktion.

5. Transplantatmaterial zur Verwendung bei der Behandlung einer Herzerkrankung gemäß einem der Ansprüche 1 bis 4, wobei die Adipozyten durch die Induzierung der Differenzierung aus Fettgewebe stammender Fibroblasten erhalten werden.

6. Transplantatmaterial zur Verwendung bei der Behandlung einer Herzerkrankung gemäß einem der Ansprüche 1 bis 5, umfassend mehrere Zellschichten in einem geschichteten Zustand.

7. Transplantatmaterial zur Verwendung bei der Behandlung einer Herzerkrankung gemäß einem der Ansprüche 1 bis 6, wobei mindestens eine der Zellschichten ferner mindestens eine umfasst, die ausgewählt ist aus der Gruppe bestehend aus Myoblasten und mesenchymalen Stammzellen.

8. Transplantatmaterial zur Verwendung bei der Behandlung einer Herzerkrankung gemäß Anspruch 7, wobei der Myoblast ein Skelettmyoblast ist.

9. Transplantatmaterial zur Verwendung bei der Behandlung einer Herzerkrankung gemäß Anspruch 7 oder 8, wobei die mesenchymale Stammzelle aus Fettgewebe stammt.

10. Transplantatmaterial zur Verwendung bei der Behandlung einer Herzerkrankung gemäß einem der Ansprüche 1 bis 9, wobei das Transplantatmaterial bei einem Individuum, von dem die Adipozyten stammen, angewendet wird.

11. Transplantatmaterial zur Verwendung bei der Behandlung einer Herzerkrankung gemäß einem der Ansprüche 1 bis 10, ferner umfassend ein Cytokin oder ein Wachstumsfaktor.

12. Transplantatmaterial zur Verwendung bei der Behandlung einer Herzerkrankung gemäß einem der Ansprüche 1 bis 11, wobei die Herzerkrankung mindestens eine Erkrankung oder Störung ist, die ausgewählt ist aus der Gruppe bestehend aus Herzversagen, ischämischer Herzerkrankung, Herzinfarkt, Kardiomyopathie, Myokarditis, hypertropher Kardiomyopathie, dilatativer Phase hypertropher Kardiomyopathie und dilatativer Kardiomyopathie.

**13.** Verfahren zur Herstellung eines Transplantatmaterials zur Behandlung von Herzerkrankungen, umfassend eine Zellschicht, die Adipozyten enthält, ohne Verwendung eines Gerüsts, wobei das Verfahren die Schritte umfasst:

a) Züchten einer Zellgruppe, die Adipozyten auf einem Zellkulturträger enthält, der mit einem temperaturempfindlichen Polymer beschichtet ist, dessen obere kritische Löslichkeitstemperatur oder untere kritische Löslichkeitstemperatur in Wasser 0 bis 80°C ist, unter Zellkulturbedingungen;
b) Einstellen der Temperatur der Kulturlösung auf die obere kritische Löslichkeitstemperatur oder darüber oder die untere kritische Löslichkeitstemperatur oder darunter; und
c) Ablösen der Zellgruppe als Zellschicht von dem Zellkulturträger.

**14.** Verfahren gemäß Anspruch 13, ferner umfassend den Schritt d) des Hinzufügens einer Ascorbinsäure oder eines Derivats davon zu der Kulturlösung vor Schritt c).

**15.** Verfahren gemäß Anspruch 13 oder 14, das keinen Schritt zur Proteasebehandlung umfasst.

**16.** Verfahren gemäß einem der Ansprüche 13 bis 15, wobei das temperaturempfindliche Polymer ein Poly(N-isopropylacrylamid) ist.

**Revendications**

**1.** Matériau de greffe à utiliser dans le traitement d'une cardiopathie comprenant une feuille de cellules contenant des adipocytes différenciés et détachée d'un support de culture cellulaire.

**2.** Matériau de greffe à utiliser dans le traitement d'une cardiopathie selon la revendication 1, dans lequel la feuille de cellules a une épaisseur de 50 $\mu$m ou plus.

**3.** Matériau de greffe à utiliser dans le traitement d'une cardiopathie selon la revendication 1 ou 2, dans lequel la feuille de cellules a une capacité de sécrétion d'adiponectine de 3 x $10^{-14}$ g/cellule ou plus par jour.

**4.** Matériau de greffe à utiliser dans le traitement d'une cardiopathie selon l'une quelconque des revendications 1 à 3, dans lequel la feuille de cellules a au moins une fonction sélectionnée dans le groupe constitué par la fonction d'inhibition des fibroblastes, la fonction angiogénique, la fonction d'inhibition de l'apoptose et la fonction anti-inflammatoire.

**5.** Matériau de greffe à utiliser dans le traitement d'une cardiopathie selon l'une quelconque des revendications 1 à 4, dans lequel les adipocytes sont obtenus en induisant une différenciation des fibroblastes dérivés du tissu adipeux.

**6.** Matériau de greffe à utiliser dans le traitement d'une cardiopathie selon l'une quelconque des revendications 1 à 5, comprenant de multiples feuilles de cellules dans un état feuilleté.

**7.** Matériau de greffe à utiliser dans le traitement d'une cardiopathie selon l'une quelconque des revendications 1 à 6, dans lequel au moins une des feuilles de cellules comprend en outre au moins l'un sélectionné dans le groupe constitué par les myoblastes et les cellules souches mésenchymateuses.

**8.** Matériau de greffe à utiliser dans le traitement d'une cardiopathie selon la revendication 7, dans lequel le myoblaste est un myoblaste squelettique.

**9.** Matériau de greffe à utiliser dans le traitement d'une cardiopathie selon la revendication 7 ou 8, dans lequel la cellule souche mésenchymateuse est dérivée du tissu adipeux.

**10.** Matériau de greffe à utiliser dans le traitement d'une cardiopathie selon l'une quelconque des revendications 1 à 9, dans lequel le matériau de greffe est appliqué sur un individu dont sont dérivés les adipocytes.

**11.** Matériau de greffe à utiliser dans le traitement d'une cardiopathie selon l'une quelconque des revendications 1 à 10, comprenant en outre une cytokine ou un facteur de croissance.

**12.** Matériau de greffe à utiliser dans le traitement d'une cardiopathie selon l'une quelconque des revendications 1 à 11, dans lequel la cardiopathie est au moins une maladie ou un trouble sélectionné dans le groupe constitué par une insuffisance cardiaque, une cardiopathie ischémique, un infarctus du myocarde, une cardiomyopathie, une myocardite, une cardiomyopathie hypertrophique, une cardiomyopathie hypertrophique en phase dilatée et une cardiomyopathie dilatée.

13. Procédé de production d'un matériau de greffe pour le traitement d'une cardiopathie comprenant une feuille de cellules contenant des adipocytes, sans utiliser de greffon de support, le procédé comprenant les étapes consistant à :

a) cultiver un groupe de cellules contenant des adipocytes sur un support de culture cellulaire revêtu d'un polymère réagissant à la température dont la température de solution critique supérieure ou la température de solution critique inférieure dans l'eau est de 0 à 80 °C, dans une solution de culture ;
b) amener la température de la solution de culture à la température de solution critique supérieure ou à une température plus haute ou à la température de solution critique inférieure ou à une température plus basse ; et
c) détacher du support de culture cellulaire le groupe de cellules en tant que feuille de cellules.

14. Procédé selon la revendication 13, comprenant en outre une étape d) d'addition d'un acide ascorbique ou d'un dérivé de celui-ci à la solution de culture avant l'étape c).

15. Procédé selon la revendication 13 ou 14, ne comprenant aucune étape de traitement par protéases.

16. Procédé selon l'une quelconque des revendications 13 à 15, dans lequel le polymère réagissant à la température est le poly(N-isopropylacrylamide).

Fig. 1

Fig. 2

Fig. 3

Fig. 4

**%infarct size**

**# of TUNEL(+) at border zone**

Fig. 5

**# of CD11b(+) at border zone**

**TNF-α**

**MCP-1**

Fig. 6

Fig. 7

Fig. 8

Fig. 9

**A**

W          K          C

**B**

Infarction

**C**

TNFa

**D**

CD11b

Fig. 10

Fig 10

**EP 2 510 956 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H02211865 A
- JP 2002008387 W
- JP 2005011524 W
- JP 2006080434 W

**Non-patent literature cited in the description**

- HO KK ; ANDERSON KM ; KANNEL WB ; GROSS-MAN W ; LEVY D. *Circulation,* 1993, vol. 88, 107-115
- RYAN TJ ; ANTMAN EM ; BROOKS NH ; CALIFF RM ; HILLIS LD ; HIRATZKA LF ; RAPAPORT E ; RIEGEL B ; RUSSELL RO ; SMITH EE III. *J Am Coll Cardiol.,* 1999, vol. 34, 890-911
- CORIN WJ ; GEORGE DT ; SINK JD et al. *J Thorac Cardiovasc Surg,* 1992, vol. 104, 1662-71
- KRATZ JM ; JOHNSON WS ; MUKHERJEE R et al. *J Thorac Cardiovasc Surg,* 1994, vol. 107, 868-78
- CARPENTIER A ; CHACHQUES JC. *Lancet,* 1985, vol. 8840, 1267
- HAGEGE AA ; DESNOS M ; CHACHQUES JC et al. Preliminary report: follow-up after dynamic cardiomyoplasty. *Lancet,* 1990, vol. 335, 1122-4
- LEOR J ; ETZION SA ; DAR A et al. *Circulation,* 2000, vol. 102 (III), III-56-III-61
- LI RK ; JIA ZQ ; WEISEL RD et al. *Circulation,* 1999, vol. 100 (II), II-63-II-69
- CARREL A. *J Exp Med,* 1907, vol. 9, 226-8
- CARREL A. *J Exp Med,* 1912, vol. 9, 389-92
- CALNE RY. *Transplant Proc,* 1970, vol. 2, 550
- AUCHINCLOSS. *Transplantation,* 1988, vol. 46, 1
- URETSKY BF ; MULARI S ; REDDY S et al. *Circulation,* 1987, vol. 76, 827-34
- SHIBATA, R. ; N. OUCHI ; T. MUROHARA. Adiponectin and cardiovascular disease. *Circ J,* 2009, vol. 73 (4), 608-14
- EREN P. ; CAMUS S. ; MATRONE G. et al. Adiponectinemia controls pro-angiogenic cell therapy. *Stem Cells,* 2009, vol. 27 (11), 2712-21
- HWANG H. ; KLONER RA. Improving regenerating potential of the heart after myocardial infarction: Factor-based approach. *Life Sciences,* 2010, vol. 86, 461-472